(19) Europäisches Patentamt European Patent Office Office européen des brevets

(11) EP 2 844 747 B1

(12) **EUROPEAN PATENT SPECIFICATION**

(45) Date of publication and mention of the grant of the patent:
**20.03.2019 Bulletin 2019/12**

(51) Int Cl.:
***C12N 9/00*** *(2006.01)*

(21) Application number: **13723713.7**

(22) Date of filing: **03.05.2013**

(86) International application number:
**PCT/EP2013/059313**

(87) International publication number:
**WO 2013/164477 (07.11.2013 Gazette 2013/45)**

(54) **A GLYCOSYLATED MODIFIED FLAVIN ADENINE DINUCLEOTIDE DEPENDENT GLUCOSE DEHYDROGENASE**

GLYKOLSYLIERTE MODIFIZIERTE FLAVINADENIN-DINUKLEOTID-ABHÄNGIGE GLUCOSE-DEHYDROGENASE

GLUCOSE DÉSHYDROGÉNASE DÉPENDANT D'UN FLAVIN-ADÉNINE-DINUCLÉOTIDE MODIFIÉ GLYCOSYLÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.05.2012 EP 12166703**

(43) Date of publication of application:
**11.03.2015 Bulletin 2015/11**

(73) Proprietors:
• **Roche Diabetes Care GmbH**
**68305 Mannheim (DE)**
Designated Contracting States:
**DE**
• **F. Hoffmann-La Roche AG**
**4070 Basel (CH)**
Designated Contracting States:
**AL AT BE BG CH CY CZ DK EE ES FI FR GB GR HR HU IE IS IT LI LT LU LV MC MK MT NL NO PL PT RO RS SE SI SK SM TR**

(72) Inventors:
• **DÜFEL, Hartmut**
**82444 Schlehdorf (DE)**
• **MEIER, Thomas**
**81373 Munich (DE)**
• **TACKE, Michael**
**80689 Munich (DE)**

(74) Representative: **Kilger, Ute**
**Boehmert & Boehmert**
**Anwaltspartnerschaft mbB**
**Pettenkoferstrasse 22**
**80336 München (DE)**

(56) References cited:
**EP-A1- 2 241 621     WO-A1-2008/102639**
**WO-A1-2009/119728     JP-A- 2010 051 312**
**JP-A- 2010 057 427     US-A1- 2008 220 460**
**US-A1- 2011 045 513**

• **MUHAMMAD NADEEM ZAFAR ET AL: "Characterization of different FAD-dependent glucose dehydrogenases for possible use in glucose-based biosensors and biofuel cells", ANALYTICAL AND BIOANALYTICAL CHEMISTRY, SPRINGER, BERLIN, DE, vol. 402, no. 6, 6 January 2012 (2012-01-06), pages 2069-2077, XP035012680, ISSN: 1618-2650, DOI: 10.1007/S00216-011-5650-7**
• **CHRISTOPH SYGMUND ET AL: "Heterologous overexpression of Glomerella cingulata FAD-dependent glucose dehydrogenase in Escherichia coli and Pichia pastoris", MICROBIAL CELL FACTORIES, BIOMED CENTRAL, LONDON, NL, vol. 10, no. 1, 12 December 2011 (2011-12-12), page 106, XP021093664, ISSN: 1475-2859, DOI: 10.1186/1475-2859-10-106**

**Description**

**Brief description of the invention**

Subject matter of the present invention is a modified flavin adenine dinucleotide dependent glucose dehydrogenase (FAD-GDH) that is glycosylated, wherein at least one of the asparagine residues selected from the group consisting of N2; N168 and N346 according to the mature *Aspergillus oryzae* FAD-GDH wild-type sequence SEQ ID No: 2 (Wild type) has been substituted by one or more amino acids not suitable for glycosylation and thereby eliminating or inactivating, respectively, a potential glycosylation site at this position.

Particularly, subject matter of the present invention is a modified flavin adenine dinucleotide dependent glucose dehydrogenase (FAD-GDH) that is glycosylated wherein:

- (a) The modified flavin adenine dinucleotide dependent glucose dehydrogenase having SEQ ID No: 2 is glycosylated, and wherein at least one of the asparagine residues selected from the group consisting of N2; N168 and N346 according to the mature *Aspergillus oryzae* FAD-GDH wild-type sequence SEQ ID No: 2 (Wild type) has been substituted by one or more amino acids not suitable for glycosylation and thereby eliminating or inactivating, respectively, a potential glycosylation site at this position, and
- (b) The modified flavin adenine dinucleotide dependent glucose dehydrogenase that is glycosylated exhibits 80% amino acid sequence identity or more to a modified flavin adenine dinucleotide dependent glucose dehydrogenase consisting of SEQ ID No: 2, wherein at least one of the asparagine residues selected from the group consisting of N2; N168 and N346 according to the mature *Aspergillus oryzae* FAD-GDH wild-type sequence of SEQ ID No: 2 has been substituted by one or more amino acids not suitable for glycosylation and thereby eliminating or inactivating, respectively, a potential glycosylation site at this position and wherein said asparagine residue has been substituted by S, P, SP or D, and
- (c) an active fragment of the modified flavin adenine dinucleotide dependent glucose dehydrogenase according to the preceding two paragraphs provided that in the FAD-GDH according to (b) or in said active fragment said substitution(s) eliminating or inactivating said potential glycosylation site(s) is/are preserved when compared to the modified flavin adenine dinucleotide dependent glucose dehydrogenase according to (a) and provided that the flavin adenine dinucleotide dependent glucose dehydrogenase according to (b) or said active fragment exhibits at least 80% of the enzyme activity of the FAD-GDH according (a) and exhibits at least 80% of the temperature stability under dry conditions of the FAD-GDH according to (a), wherein the expression "exhibits temperature stability under dry conditions" means residual activity of the lyophilizated modified flavin adenine dinucleotide dependent glucose dehydrogenase (FAD-GDH) itself and when comprised in a lyophilizated composition, calculated and compared to the unstressed lyophilizate after: 1) lyophilization and incubation of the lyophilized enzyme at 80°C for 8 days over molecular sieve (3A, MS551, Grace).

The present invention further relates to a process for producing the same, nucleic acids encoding said enzymes, vectors, host cells, a method of detecting, determining or measuring glucose in a sample using said enzyme and devices comprising said enzyme.

**Field of the Invention**

Self-monitoring of blood glucose is important for people with diabetes to be aware of their usual glucose levels and to use them for their treatment. Enzymes having glucose substrates are employed as sensors for blood glucose self-monitoring. Examples of such enzymes include glucose oxidase (EC 1.1.3.4). Glucose oxidase has the advantages of being highly specific to glucose and having high heat stability. For this reason, it has been used as an enzyme in blood glucose sensors. The first announcement of such properties goes back to as long as 40 years ago. In blood glucose sensors that utilize glucose oxidase, the blood glucose level is measured when electrons generated in the process of converting glucose to D-glucono-d-lactone by oxidization are conducted to an electrode via a mediator. However, glucose oxidase poses a problem in that it tends to transfer protons produced by the reaction to oxygen, causing dissolved oxygen to adversely affect the measured values.

To solve such a problem, for example, NAD(P) dependent glucose dehydrogenase (EC 1.1.1.47) or pyrroloquinoline quinone (hereinafter also referred to as "PQQ" in the specification) dependent glucose dehydrogenase (EC1.1.5.2 (former EC1.1.99.17)) is used as an enzyme in blood glucose sensors. They have the advantage of being free from the influence of dissolved oxygen. However, the former, *i.e.,* NAD(P) dependent glucose dehydrogenase (hereinafter also referred to as "NADGDH" in the specification) has poor stability and is cumbersome, requiring the addition of a coenzyme. The latter, i.e., PQQ dependent glucose dehydrogenase (hereinafter also referred to as "PQQGDH" in the specification), has the drawbacks of having poor substrate specificity and reacting to saccharides other than glucose, such as maltose and lactose, thereby deteriorating the accuracy of the measurement values.

Further, WO-A1 2004/058958 discloses *Aspergillus*-flavin-bound glucose dehydrogenase. Since the activity of this enzyme on xylose is only 10% of that on glucose, in the case of measuring the blood glucose level of a person who is taking a xylose tolerance test, the accuracy of the measured value may be impaired. The enzyme has a residual activity ratio of about 89% after treatment at 50°C for 15 minutes, thereby exhibiting good heat stability. WO-A1 2006/101239 discloses the gene sequence and amino acid sequence of the enzyme.

A modified flavin adenine dinucleotide dependent glucose dehydrogenase (FAD-GDH) with improved heat stability in liquid than FAD-GDH derived from wild-type FAD-GDH, the modified FAD-GDH being derived from preferably an eukaryote, more preferably a filamentous fungus, and furthermore preferably an *Aspergillus* fungus, and, for example, those having a primary structure with at least one amino acid substituted, deleted, inserted or added to FAD-GDH is provided by US-B2 7,662,600.

US 2008/220460 A1 describes a modified FAD-GDH derived from Aspergillus fungus, e.g. *Aspergillus oryzae* or *Aspergillus terreus* having improved heat stability as compared to wild-type FAD-GDHs. US 2008/220460 A1 focuses only on a modified FAD-GDH produced by gene recombination in *E.coli.* Hence, said FAD-GDHs are non-glycosylated enzyme variants which were also only screened under liquid conditions in US 2008/220460 A1. Therefore, this document is silent about specific modifications to the nucleotide sequence to obtain FAD-GDH variants that are glycosylated, and having improved heat stability under dry conditions in result of elimination or inactivation of a potential glycosylation site.

For some uses of the FAD-GDH the heat stability under dry conditions is of special importance. For instance, in case of test strips for blood-glucose measurements the enzyme properties of FAD-GDH under dry chemistry needs to be improved.

It was a subject of the present invention to provide an improved flavin adenine dinucleotide dependent glucose dehydrogenase. It was of special interest to provide such an improved enzyme in view of the temperature stability under dry conditions.

## Detailed Description of the Invention

Subject matter of the present invention is a modified flavin adenine dinucleotide dependent glucose dehydrogenase that is glycosylated, selected from the group consisting of

(a) a modified flavin adenine dinucleotide dependent glucose dehydrogenase having SEQ ID No: 2 that is glycosylated, but wherein at least one of the asparagine residues selected from the group consisting of N2; N168 and N346 according to the mature *Aspergillus oryzae* FAD-GDH wild-type sequence SEQ ID No: 2 (Wild type) has been substituted by one or more amino acids not suitable for glycosylation and thereby eliminating or inactivating, respectively, a potential glycosylation site at this position and

(b) a modified flavin adenine dinucleotide dependent glucose dehydrogenase that is glycosylated that exhibits 80% amino acid sequence identity or more to a modified flavin adenine dinucleotide dependent glucose dehydrogenase consisting of SEQ ID No: 2, but wherein at least one of the asparagine residues selected from the group consisting of N2; N168 and N346 according to the mature *Aspergillus oryzae* FAD-GDH wild-type sequence according to SEQ ID No: 2 has been substituted by one or more amino acids not suitable for glycosylation and thereby eliminating or inactivating, respectively, a potential glycosylation site at this position and wherein said asparagine residue has been substituted by S, P, SP or D, and

(c) an active fragment of a modified flavin adenine dinucleotide dependent glucose dehydrogenase according to (a) or (b) provided that in the FAD-GDH according to (b) or the fragment according to (c) said substitution(s) eliminating or inactivating said potential glycosylation site(s) is/are preserved when compared to the modified flavin adenine dinucleotide dependent glucose dehydrogenase according to (a) and provided that the flavin adenine dinucleotide dependent glucose dehydrogenase according to (b) or the fragment according to (c) exhibits at least 80% of the enzyme activity of the FAD-GDH according (a) and exhibits at least 80% of the temperature stability under dry conditions of the FAD-GDH according to (a), wherein the expression "exhibits temperature stability under dry conditions" means residual activity of the lyophilized modified flavin adenine dinucleotide dependent glucose dehydrogenase (FAD-GDH) itself and when comprised in a lyophilized composition, calculated and compared to the unstressed lyophilizate after lyophilization and incubation of the lyophilized enzyme at 80°C for 8 days over molecular sieve (3A, MS551, Grace).

It shall be understood that throughout the specification the percental values are not absolute values, but rather relative values encompassing a certain margin of error of ± 5%. The person skilled in the art is aware that such a variation is evident in view of the subject matter of the present invention.

The term "active fragment(s) thereof' or synonymous "functional fragment(s) thereof" refer to any modified flavin adenine dinucleotide dependent glucose dehydrogenase pursuant to the invention, whereby at least one amino acid is missing in the corresponding sequence according to SEQ ID No. 3 to 6, provided that such fragments still exhibit the essential properties as regards enzyme activity and the improved temperature stability under dry conditions in the sense of the present invention, wherein an active fragment of a modified flavin adenine dinucleotide dependent glucose dehydrogenase exhibits at least 80% of the enzyme activity of the FAD-GDH according to the present invention and exhibits at least 80% of the temperature stability under dry conditions of the FAD-GDH according to the present invention, wherein the expression "exhibits temperature stability under dry conditions" means residual activity of the lyophilized modified flavin adenine dinucleotide dependent glucose dehydrogenase (FAD-GDH) itself and when comprised in a lyophilized composition, calculated and compared to the unstressed lyophilizate after lyophilization and incubation of the lyophilized enzyme at 80°C for 8 days over molecular sieve (3A, MS551, Grace).

The modified flavin adenine dinucleotide dependent glucose dehydrogenase according to the present invention exhibits improved temperature stability under dry conditions when compared to those FAD-GDHs of the prior art.

This has been achieved by the substitution of at least one of the asparagine residues selected from the group consisting of N2; N168 and N346 according to the mature *Aspergillus oryzae* FAD-GDH wild-type sequence SEQ ID No: 2 (Wild type) by one or more amino acids not suitable for glycosylation and thereby eliminating or inactivating, respectively, a potential glycosylation site at this position. It shall be understood that also FAD-GDHs that exhibit at least 80% amino acid sequence identity (preferably 90%, more preferably 95%) to the FAD-GDHs according to the present invention as above outlined under (a) are subject matter of the present invention provided that they exhibit the same substitution(s) and exhibit essentially the same properties as an FAD-GDH according to the present invention, those essential properties being enzyme activity and the improved temperature stability under dry conditions. It shall be understood that also fragments of said FAD-GDHs according to the present invention are encompassed that exhibit the same substitution(s) and exhibit essentially the same properties as a FAD-GDH according to the present invention, those essential properties being enzyme activity and the improved temperature stability under dry conditions.

Sequence identity may be determined by the BLAST algorithm, the Basic Local Alignment Search Tool (BLAST) (Altschul, S.F. et al. 1990. J. Mol. Biol. 215:403; Altschul, S.F. et al. 1997. Nucleic Acid Res. 25:3389-3402). The above-mentioned percentages of amino acid sequence identity refer to the determination of sequence identity by said BLAST algorithm, wherein the region over which the homology is determined is the entire sequence of the modified FAD-GDH according to (a), and wherein said sequence of the FAD-GDH according to (a) is the reference sequence.

Throughout the specification and the corresponding claims the term "mature" in relation to "sequence(s)" refers to the raw sequence format of the respective protein sequence(s) without any added signaling sequences, e.g. signal peptides or equivalents thereof.

Throughout the specification the term "temperature stability" refers to the ability of the herein provided modified flavin adenine dinucleotide dependent glucose dehydrogenases (FAD-GDH) to resist changes in terms of its native biophysical and biochemical properties as its temperature changes, in particular its temperature increases. With this context, 100% temperature stability would reflect that no changes occur to the native biophysical and biochemical properties when compared to the enzyme before exposure to temperature over a certain period of time (t) with respect to particularly defined properties of the enzyme. Thus, the herein provided modified flavin adenine dinucleotide dependent glucose dehydrogenases or functional fragments thereof preserve its native enzyme activity during exposure to temperature over a certain period of time (t), and exemplarily exhibit an enzyme activity measured according to Example 3 as a specific embodiment, but not limited to.

Throughout the entire specification the term "dry condition(s)" is specifically related to the test conditions according to Example 7 as set out below, namely the respective lyophilized sample was exposed to 80°C for 8 days in presence of a drying agent (molecular sieve 3A, MS 551, Grace), but not limited to.

With the context of the entire specification and the corresponding claims the expression "exhibits temperature stability under dry condition(s)" means residual activity of the lyophilizated modified flavin adenine dinucleotide dependent glucose dehydrogenase (FAD-GDH) itself and when comprised in a lyophilized composition, calculated and compared to the unstressed lyophilizate after: 1) lyophilization and incubation of the lyophilized enzyme at 80°C for 8 days over molecular sieve (3A, MS551, Grace).

The "unstressed lyophilizate" is the part of the enzyme that has been lyophilizated but not incubated at 80°C for 8 days over molecular sieve (3A, MS551, Grace). The "unstressed lyophilizate" means that the temperature stability is determined subsequent to lyophilization. This means the unstressed lyophilizate is neither stored nor treated before determining the temperature stability. Temperature stability under dry conditions may be determined according to Example 7, but not limited to.

In another specific embodiment the invention also foresees modified flavin adenine dinucleotide dependent glucose dehydrogenases, whose sequence is a functional equivalent to sequences as provided herein under SEQ ID No. 3 to 6. In the context of the present invention a functional equivalent is an amino acid sequence molecule, which is different in at least one amino acid as provided in the sequences SEQ ID No. 3 to 6, which encodes a protein/enzyme with same or similar function, in particular in terms of enzyme activity and thermal stability under dry conditions as defined above.

In a further specific embodiment of the present invention the functional equivalent of the modified flavin adenine dinucleotide dependent glucose dehydrogenases according to the invention is an amino acid sequence homologous to the SEQ ID No. 3 to 6. In a specific embodiment of the present invention the degree or percentage of homology is at least 80, 90, 95, 99 or 100% to the SEQ ID No. 3 to 6; provided that they exhibit the same substitutions as outlined above under (a) and exhibit essentially the same properties as an FAD-GDH according to the present invention, those essential properties being enzyme activity and the improved temperature stability under dry conditions. It shall be understood that also active fragments of said FAD-GDHs according to the present invention are encompassed that exhibit the same substitutions and exhibit essentially the same properties as an FAD-GDH according to the present invention, those essential properties being enzyme activity and the improved temperature stability under dry conditions as defined above.

The same applies to functional equivalent nucleotide sequence molecules, which codes with a different nucleotide sequence to SEQ ID No. 13 or the complementary sequence thereto, for exactly the same protein/enzyme.

In a furthermore specific embodiment a functional equivalent of the nucleotide molecule as given in the SEQ ID No. 13 is a RNA molecule, which is encoded by said DNA sequence or a sequence being substantially complementary to the sequence of SEQ ID No. 13.

In the context of the present invention the term "RNA molecule" is meant to refer to a linear polymer of ribonucleotide molecules, which is single-stranded and serves as a template for protein synthesis of the herein provided modified flavin adenine dinucleotide dependent glucose dehydrogenases according to the SEQ ID No. 3 to 6.

"Drying agents" in the context of the present invention are for instance desiccants such as silica gel, calcium sulfate, calcium chloride, and molecular sieves, but not limited to.

Particularly, subject matter of the present invention is a modified flavin adenine dinucleotide dependent glucose dehydrogenase (FAD-GDH) that is glycosylated, or a FAD-GDH that exhibits at least 80% sequence homology thereto (preferably at least 90%, more preferably at least 95%) or functional fragments thereof as defined above and in the claims wherein:

- said modified flavin adenine dinucleotide dependent glucose dehydrogenase exhibits an improved temperature stability under dry conditions in comparison to glycosylated FAD-GDH according to SEQ ID No: 1, wherein said FAD-GDH according to SEQ ID No: 1 is obtainable by expression in *Aspergillus oryzae* and

- wherein at least one of the asparagine residues selected from the group consisting of N2; N168 and N346 according to the mature *Aspergillus oryzae* FAD-GDH wild-type sequence SEQ ID No: 2 (Wild type) has been substituted by one or more amino acids and thereby eliminating or inactivating, respectively, a potential glycosylation site at this position.

The modified flavin adenine dinucleotide dependent glucose dehydrogenase (FAD-GDH) is glycosylated. In one embodiment of the present invention said modified flavin adenine dinucleotide dependent glucose dehydrogenase or functional fragments thereof exhibit a more homogenous glycosylation pattern (with less molecular weight distribution) in comparison to glycosylated FAD-GDH according to SEQ ID No: 1, wherein said FAD-GDH according to SEQ ID No: 1 is obtainable by expression in *Aspergillus oryzae.* In another specific embodiment, the modified flavin adenine dinucleotide dependent glucose dehydrogenase or functional fragments thereof according to the present invention exhibit a molecular Weight Average Molecular Weight ($M_w$) of about 103 876 and a Number Average Molecular Weight ($M_n$) of about 99 901. The Weight Average Molecular Weight ($M_w$) and the Number Average Molecular Weight ($M_n$) is calculated by means of the software from the raw data of the Viscotek Triple Detektors (refraction index (RI) and Right Angle Light Scattering (RALS)). The ratio of $M_w/M_n$ is the polydispersity and gives the size distribution of the protein. Monodisperse proteins have a $M_w/M_n$ value of 1.

Thus, in one embodiment of the present invention the compositions comprising a modified flavin adenine dinucleotide dependent glucose dehydrogenase or functional fragments thereof according to the invention exhibit a molecular weight distribution of said modified flavin adenine dinucleotide dependent glucose dehydrogenase in said composition as follows: Weight Average Molecular Weight ($M_w$) of about 103 876 and a Number Average Molecular Weight ($M_n$) of about 99 901. Such compositions are another embodiment of the present invention.

Further, one embodiment of the present invention is a modified flavin adenine dinucleotide dependent glucose dehydrogenase that is glycosylated or a FAD-GDH that exhibits at least 80% sequence homology thereto, (preferably at least 90%, more preferably at least 95%) or functional fragments thereof, wherein at least one of the asparagine residues selected from the group consisting of N2; N168 and N346 according to the mature *Aspergillus oryzae* FAD-GDH wild-type sequence SEQ ID No: 2 (Wild type) has been substituted by one or more amino acids not suitable for glycosylation and thereby eliminating or inactivating, respectively, a potential glycosylation site at this position and wherein the degree of glycosylation is < 50%, preferably < 40% and more preferably < 30% and/ or the ratio of Mw/Mn is < 1,02, more preferably < 1,01 (for calculation of these values see *e.g.* Example 4).

Moreover, subject matter of the present invention is also a modified FAD-GDH or a FAD-GDH that exhibits at least 80% sequence homology thereto, (preferably at least 90%, more preferably at least 95%) or functional fragments thereof, wherein at least one of the asparagine residues selected from the group consisting of N2; N168 and N346 according to the mature *Aspergillus oryzae* FAD-GDH wild-type sequence SEQ ID No: 2 (Wild type) has been substituted by one or more amino acids and thereby eliminating or inactivating, respectively, a potential glycosylation site at this position and wherein, compositions comprising said modified FAD-GDH exhibit a molecular weight distribution with a ratio of Mw/Mn is < 1,02, more preferably with a ratio of Mw/Mn < 1,01 of said modified flavin adenine dinucleotide dependent glucose dehydrogenase in said composition and/ or wherein the degree of glycosylation of said modified FAD-GDH is < 50%, preferably < 40% and more preferably < 30%. Such compositions are another embodiment of the present invention.

The degree of glycosylation may be calculated according to the following formula:

(Mw (enzyme with glycosylation) - Mw (enzyme according to protein sequence without glycosylation))*100%. For the exemplified enzymes according to Table 3 the following may be calculated (see Example 4):

FAD-GDH variant 1 (SEQ ID No: 3):

$$(76333-61461)/61461*100\%=24\%$$

FAD-GDH (SEQ ID No: 1) from *Aspergillus:*

$$(103876-61592)/61592*100\%=69\%.$$

This means that said modified flavin adenine dinucleotide dependent glucose dehydrogenase or fragments thereof according to the present invention exhibit a more homogenous glycosylation pattern (with less molecular weight distribution) in comparison to glycosylated FAD-GDH according to SEQ ID No: 1, wherein said FAD-GDH according to SEQ ID No: 1 is obtainable by expression in *Aspergillus oryzae* and/ or said modified flavin adenine dinucleotide dependent glucose dehydrogenase or fragments thereof according to the present invention exhibit a lower degree of glycosylation when compared to FAD-GDH according to SEQ ID No: 1, wherein said FAD-GDH according to SEQ ID No: 1 is obtainable by expression in *Aspergillus oryzae.*

In one specific embodiment of the invention the residual activity of the lyophilizated modified flavin adenine dinucleotide dependent glucose dehydrogenase or functional fragments thereof calculated and compared to unstressed lyophilizate after: 1) lyophilization and incubation of the lyophilizated enzyme at 80°C for 8 days over molecular sieve (3A, MS551, Grace) is at least 80%, preferred at least 84%.

The enzymatic activity may be exemplarily determined according to Example 3 i), but not limited to.

The sugar specificity of the modified FAD-GDH or active fragments thereof according to the present invention is about the same as the sugar specificity of the glycosylated mature *Aspergillus oryzae* FAD-GDH wild-type sequence SEQ ID No: 2 (Wild type), namely for maltose < 0,5% and for galactose < 13%; see Example 3 ii) for the calculation of sugar specificity.

In a specific embodiment said at least one of the asparagine residues selected from the group consisting of N2; N168 and N346 according to the mature *Aspergillus oryzae* FAD-GDH wild-type sequence SEQ ID No: 2 (Wild type) has been substituted by one or more amino acids selected from the group comprising Ala, Arg, Asp, Cys, Gln, Glu, Gly His, Ile, Leu, Lys, Met, Phe, Pro, Ser, Thr, Trp, Tyr, and Val. In a more specific embodiment said at least one of the asparagine residues selected from the group consisting of N2; N168 and N346 according to the *Aspergillus oryzae* FAD-GDH wild-type sequence SEQ ID No: 2 (Wild type) has been substituted by one or more amino acids selected from the group comprising Arg, Asp, Gln, Glu, Gly His, Lys, Met, Pro, Ser, and Thr.

In another specific embodiment of the invention a modified flavin adenine dinucleotide dependent glucose dehydrogenase or a FAD-GDH that exhibits at least 80% sequence homology thereto, (preferably at least 90%, more preferably at least 95%) or functional fragments thereof is provided, wherein only one asparagine of the residues selected from the group consisting of N2; N168 and N346 has been substituted by one or more amino acids, which lead to an inactivation (or deletion) of the corresponding glycosylation target site.

In another specific embodiment of the invention a modified flavin adenine dinucleotide dependent glucose dehydrogenase or a FAD-GDH that exhibits at least 80% sequence homology thereto, (preferably at least 90%, more preferably at least 95%) or functional fragments thereof is provided according to the above embodiments, wherein said asparagine residue has been substituted by S, P, SP or D.

According to the present invention a specific embodiment is a modified flavin adenine dinucleotide dependent glucose dehydrogenase or a FAD-GDH that exhibits at least 80% sequence homology thereto, (preferably at least 90%, more preferably at least 95%) or functional fragments thereof, wherein said modified flavin adenine dinucleotide dependent dehydrogenase derived from mature *Aspergillus oryzae* FAD-GDH wild-type sequence SEQ ID No: 2 (Wild type) has at least one of the following substitutions N2S, N168P, N168SP, and N346D. In this context N168SP means that an asparagine residue at the position 168 according to SEQ ID No: 2 (Wild type) has been substituted by serine (S) and proline (P).

An especially specific embodiment of the present invention is the provision of a modified flavin adenine dinucleotide dependent glucose dehydrogenase or a FAD-GDH that exhibits at least 80% sequence homology thereto, (preferably at least 90%, more preferably at least 95 %) or functional fragments thereof, wherein said modified flavin adenine dinucleotide dependent dehydrogenase derived from mature *Aspergillus oryzae* FAD-GDH wild-type sequence SEQ ID No: 2 (Wild type) has one or more of the following substitutions: N2S, N168P, N168SP, and N346D.

It is another specific embodiment of the invention that said modified flavin adenine dinucleotide dependent dehydrogenase or a FAD-GDH that exhibits at least 80% sequence homology thereto, (preferably at least 90%, more preferably at least 95%) or functional fragments thereof has only one of the following substitutions N2S, N168P, N168SP, and N346D.

A person skilled in the art understands that said modified FAD-GDH may have or may have not further modifications different from the before-mentioned substitutions. The same applies to all mentioned above and all hereinafter mentioned modified FAD-GDHs according to the present invention.

Another especially specific embodiment of the present invention is the provision of a modified flavin adenine dinucleotide dependent glucose dehydrogenase or a FAD-GDH that exhibits at least 80% sequence homology thereto, (preferably at least 90%, more preferably at least 95%) or functional fragments thereof, wherein said modified flavin adenine dinucleotide dependent dehydrogenase derived from mature *Aspergillus oryzae* FAD-GDH wild-type sequence SEQ ID No: 2 (Wild type) has the particular substitution N2S according to SEQ ID No: 3.

Another specific embodiment of the present invention is the provision of a modified flavin adenine dinucleotide dependent glucose dehydrogenase or a FAD-GDH that exhibits at least 80% sequence homology thereto, (preferably at least 90%, more preferably at least 95%) or functional fragments thereof, wherein said modified flavin adenine dinucleotide dependent dehydrogenase derived from mature *Aspergillus oryzae* FAD-GDH wild-type sequence SEQ ID No: 2 (Wild type) has the substitution N168P according to SEQ ID No: 4.

Another specific embodiment of the present invention is the provision of a modified flavin adenine dinucleotide dependent glucose dehydrogenase or a FAD-GDH that exhibits at least 80% sequence homology thereto, (preferably at least 90%, more preferably at least 95%) or functional fragments thereof, wherein said modified flavin adenine dinucleotide dependent dehydrogenase derived from mature *Aspergillus oryzae* FAD-GDH wild-type sequence SEQ ID No: 2 (Wild type) has the following substitution N168SP according to SEQ ID No: 5.

Another specific embodiment of the present invention is the provision of a modified flavin adenine dinucleotide dependent glucose dehydrogenase or a FAD-GDH that exhibits at least 80% sequence homology thereto, (preferably at least 90%, more preferably at least 95%) or functional fragments thereof, wherein said modified flavin adenine dinucleotide dependent dehydrogenase derived from mature *Aspergillus oryzae* FAD-GDH wild-type sequence SEQ ID No: 2 (Wild type) has the following substitution N346D according to SEQ ID No: 6.

Encompassed by the present invention are also enzymes that exhibit at least 80% sequence homology, more preferred 90%, most preferred 95% sequence homology to all above mentioned enzymes, provided that in particular the characteristic enzyme activity and temperature stability, especially under dry conditions, remain essentially the same as mentioned throughout the specification for the provided modified flavin adenine dinucleotide dependent glucose dehydrogenases according to the sequences SEQ ID No. 3 to 6, when said enzyme activity is exemplarily determined according to Example 3 i), but not limited to.

Another specific embodiment of the present invention is an isolated polynucleotide encoding a modified flavin adenine dinucleotide dependent glucose dehydrogenase or a functional fragment thereof according to the present invention with the proviso that said isolated polynucleotide is not encoding for a modified flavin adenine dinucleotide dependent glucose dehydrogenase or an active fragment thereof derived from *Aspergillus oryzae* FAD-GDH wild-type sequence SEQ ID No: 2 (Wild type) that has a single substitution selected from the group consisting of N168K, N168P, N168Y, or N168W. The isolated polynucleotide may be a DNA or RNA molecule or a corresponding gene thereof encoding for the following sequences as explicitly listed in the section Sequence Listing:

SEQ ID No: 3, wherein the asparagine residue on position 2 is replaced by a serine residue.

SEQ ID No: 5, wherein the asparagine residue on position 168 is replaced by two amino acids, namely a serine and a proline residue.

SEQ ID No: 6, wherein the asparagine residue on position 346 is replaced by an aspartic acid residue.

Another specific embodiment of the present invention is a composition comprising one or more of the modified FAD-GDHs provided by the present invention without any restrictions to the related sequences or homogenous sequences thereof.

Another specific embodiment of the present invention is a composition comprising one or more of the isolated polynucleotides that are a DNA or RNA molecule or a corresponding gene provided by the present invention. However, in accordance with the invention said isolated polynucleotide is not encoding for a modified flavin adenine dinucleotide dependent glucose dehydrogenase or an active fragment thereof derived from *Aspergillus oryzae* FAD-GDH wild-type sequence SEQ ID No: 2 (Wild type) that has a single substitution selected from the group consisting of N168K, N168P, N168Y, or N168W.

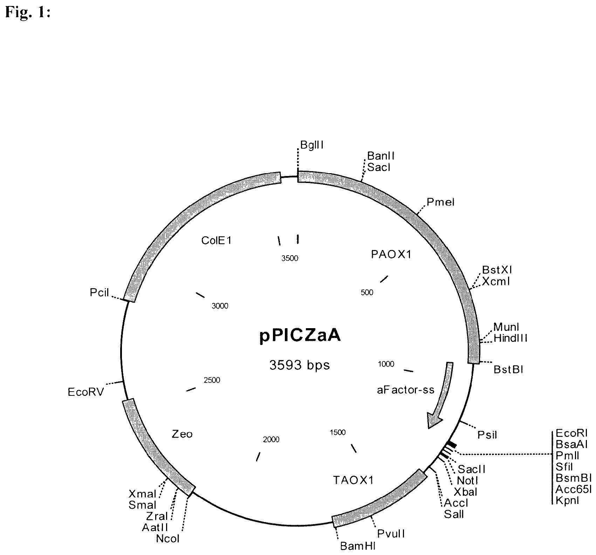

Another embodiment of the present invention is an expression vector containing the isolated polynucleotide according to the present invention. However, in accordance with the invention said isolated polynucleotide is not encoding for a modified flavin adenine dinucleotide dependent glucose dehydrogenase or an active fragment thereof derived from *Aspergillus oryzae* FAD-GDH wild-type sequence SEQ ID No: 2 (Wild type) that has a single substitution selected from the group consisting of N168K, N168P, N168Y, or N168W. Said expression vector may be operably linked to a promoter sequence capable of directing its expression in a host cell. The specific vector in this study was expression vector pPICZαA (Invitrogen). This plasmid allows replication in *E. coli* (pUC origin of replication) for cloning of the expression construct as well as recombinant gene expression in *Pichia pastoris* by use of the AOX1 promotor/AOX1 terminator sequences (further details see Figure 1).

Expression vectors useful in the present invention may typically contain an origin of replication (ori), an antibiotic resistance for selection, a promoter for expression and the whole or part of the modified FAD-GDH gene variant. However, in accordance with the invention said gene variant is not encoding for a modified flavin adenine dinucleotide dependent glucose dehydrogenase or an active fragment thereof derived from *Aspergillus oryzae* FAD-GDH wild-type sequence SEQ ID No: 2 (Wild type) that has a single substitution selected from the group consisting of N168K, N168P, N168Y, or N168W.

The expression vectors may also include other DNA sequences known in the art, like signal sequences (for a better folding, transportation into the periplasm or secretion) inducers for a better modulation of the expression, or cleavage sites for cloning. The characteristics of the selected expression vector must be compatible to the host cell which is to be employed. Suitable origins of replications like the CoIE1 plasmid replication origin can be used. Suitable promoters include, for example, *lac* and *trp.* It is also desirable that the expression vector includes a sequence coding for a selection marker like an antibiotic resistance gene. As selectable markers, ampicillin resistance, or kanamycin resistance may be conveniently employed. All of these materials are known in the art and are commercially available.

Suitable expression vectors containing the desired coding and control sequences may be constructed using standard recombinant DNA techniques known in the art, many of which are described in Sambrook et al., in "Molecular Cloning: A Laboratory Manual" (1989), Cold Spring Habor, NY Cold Spring Habor Laboratory Press.

Another embodiment of the present invention is a host cell comprising the expression vector according to the present invention. The person skilled in the art understands that in accordance with the invention said host cell must be suitable for glycosylation. Therefore, in accordance with the invention said host cell - as a prerequisite - is having endogenous glycosylating enzymes, particularly for *N*-linked glycosylation.

Accordingly, another embodiment of the present invention is a host cell comprising the expression vector according to the present invention with the proviso that said host cell is capable of glycosylation by having endogenous glycosylating enzymes, particularly for *N*-linked glycosylation.

Another specific embodiment of the invention is a host cell comprising the expression vector according to the present invention with the proviso that said host cell is capable of glycosylation, particularly for *N*-linked glycosylation by having endogenous glycosylating enzymes, and said host cell is not an *E.coli* strain.

"Glycosylating enzymes" catalyze the reaction in which a carbohydrate, i.e. a glycosyl donor, is attached to a hydroxyl or other functional group of another molecule. This is an enzymatic process that attaches glycans to proteins, lipids, or other organic molecules. Glycosylation is a form of co-translational and post-translational modification. The majority of proteins synthesized in the rough ER undergo glycosylation. It is an enzyme-directed site-specific process, as opposed to the non-enzymatic chemical reaction of "glycation".

In accordance with the invention a "host cell capable for glycosylation, particularly for *N*-linked glycosylation by having endogenous glycosylating enzymes" is a host cell derived from but not limited to *Aspergillus niger, Aspergillus sojae, Aspergillus oryzae, Pichia pastoris, Saccharomyces cerevisiae, Hansenula polymorpha,* Suitable *Pichia* host cells include - without being limited thereto - *P. pastoris* X33 or *P. pastoris* KM71H available from Invitrogen (5791 Van Allan Way, Carlsbad, CA 92008, USA).

The person skilled in the art is aware that native *E.coli* strains generally lack a glycosylation system and therefore do not express glycosylated proteins. Only in case a glycosylation system has been genetically engineered into *E.coli* (e.g. the *N*-linked glycosylation system of *Campylobacter jejuni),* they are enabled to produce glycoproteins.

The host cells preferably contain an expression vector that comprises all or part of one of the DNA sequences coding for a modified FAD-GDH enzyme having one or more mutations according to the present invention.

The enzyme according to SEQ ID No.: 01 may be expressed in *Aspergillus oryzae* and may be expressed as described in e.g. Japanese Patent JP 2010/239969, US-A1 20090259024, US-A 20090155848, US-A1 020080090278; US-A1 20080020426, US-A1 20080014612, US-A1 20080014611, US-A1 20080003628, US-B2 7,871,805, US-B2 7,741,100, US-B2 7,655,130, US-B2 7,553,649 and US-B2 7,494,794.

The recombinant production of the modified flavin adenine dinucleotide dependent glucose dehydrogenase according to the present invention may be conducted in hosts known in the art. Suitable hosts may be selected from strains of filamentous fungi as *e.g. Aspergillus niger, Aspergillus sojae* and *Aspergillus oryzae.* Suitable hosts may be selected from strains of yeast as e.g. *Pichia pastoris, Saccharomyces cerevisiae* and *Hansenula polymorpha.* Suitable *Pichia* host cells include, for example, *P. pastoris* X33 or *P. pastoris* KM71H available from Invitrogen (5791 Van Allan Way, Carlsbad, CA 92008, USA).

It is understood by the person skilled in the art that the modified FAD-GDH pursuant to the invention may have or may have not further modifications that are different from the before-mentioned modification(s).

In a specific embodiment the modified FAD-GDH or functional fragments thereof according to the present invention are obtainable by expression of a polynucleotide encoding said modified FAD-GDH according to the present invention in yeast, in particular in *Pichia pastoris.*

Expression vectors may be introduced into host cells by various methods known in the art. For example, transformation of host cells with expression vectors can be carried out by polyethylene glycol mediated protoplast transformation method (Sambrook *et al.* 1989, supra). However, other methods for introducing expression vectors into host cells, for example, electroporation, ballistic DNA injection, or protoplast fusion, can also be employed.

Suitable host cells may be but are not limited to yeast cells like *Pichia pastoris, Saccharomyces cerevisiae* or *Hansenula polymorpha* or filamentous fungi like *Aspergillus niger* or *Aspergillus sojae.*

A specific embodiment of the present invention is a host cell comprising the expression vector according the present invention wherein said host cell is from yeast, in particular *Pichia pastoris.*

Once an expression vector containing a modified FAD-GDH variant has been introduced into an appropriate host cell, the host cell may be cultured under conditions permitting expression of the desired modified FAD-GDH variants. Host cells containing the desired expression vector with the DNA sequence coding for all or part of the modified FAD-GDH can be easily identified by *i.e.* antibiotic selection or complementation of auxotrophic mutants and selection from minimal medium (J. Sambrook, D. W. Russell: "Molecular Cloning: a laboratory manual", 3rd edition, Cold Spring Harbor, New York (2001)). The expression of the modified FAD-GDH variants can be identified by different methods like measuring production of FAD-GDH mRNA transcripts, detection of the gene product immunologically or detection of the enzymatic activity of the gene product. Preferably, an enzymatic assay is applied.

It should be understood by the person skilled in the art that not all expression vectors and DNA regulatory sequences would function equally well to express the DNA sequences of the present invention. Neither will all host cells function equally well with the same expression system. However, one of ordinary skill in the art will make an appropriate selection among the expression vectors, DNA regulatory sequences, and host cells using the guidance provided herein without undue experimentation. In this regard it should be understood that another specific embodiment of the invention provides for suitable host cells comprising the expression vector according to the invention, including an isolated polynucleotide that is encoding for a modified flavin adenine dinucleotide dependent glucose dehydrogenase or an active fragment thereof derived from *Aspergillus oryzae* FAD-GDH wild-type sequence SEQ ID No: 2 (Wild type) that has a single substitution selected from the group consisting of N168K, N168P, N168Y, or N168W, with the proviso that said host cells are capable of glycosylation, particularly for *N*-linked glycosylation by having endogenous glycosylating enzymes, and said host cells are not *E.coli* strains.

Another embodiment of the present invention is a process for producing a modified flavin adenine dinucleotide dependent glucose dehydrogenase or functional fragments thereof, the process comprising culturing the transformant according to the present invention.

The invention also relates to a process for producing FAD-GDH variants of the current invention comprising culturing a host cell of the invention under conditions suitable for production of the modified FAD-GDH of the invention. For bacterial host cells, typical culture conditions are liquid medium containing carbon and nitrogen sources, the appropriate antibiotic and induction agent (depending on the used expression vector). Typical appropriate antibiotics include ampicillin, kanamycin, chloramphenicol, tetracycline (as well as Zeomycin for *P. pastoris*) and the like. Typical induction agents include IPTG, glucose, lactose (for *E. coli*) as well as methanol for *P. pastoris* and the like.

It is another embodiment of the invention that the polypeptides of the present invention are obtained by production in host cells expressing a DNA sequence coding for the modified FAD-GDH. The polypeptides of the present invention may also be obtained by *in vitro* translation of the mRNA encoded by a DNA sequence coding for the modified FAD-GDH. For example, the DNA sequences may be synthesized as described above and inserted into a suitable expression vector, which in turn may be used in an *in vitro* transcription/ translation system.

An expression vector comprising an isolated polynucleotide as defined and described above operably linked to a promoter sequence capable of promoting its expression in a cell-free peptide synthesis system represents another specific embodiment of the present invention.

The polypeptides produced *e.g.* by procedures as describe above, may then be isolated and purified using various routine protein purification techniques. For example, chromatographic procedures such as ion exchange chromatography, gel filtration chromatography and affinity chromatography may be employed.

Another subject of the present invention is accordingly a modified flavin adenine dinucleotide dependent glucose dehydrogenase or functional fragments thereof obtainable by the process of the present invention.

One of the major applications of the improved FAD-GDH variants of this invention is for the use in test strips to monitor the blood-glucose level in *ex vivo* samples of diabetic patients. Of course many kinds of samples may be investigated. Bodily fluids like serum, plasma, intestinal fluid or urine are preferred sources for such samples.

The FAD-GDH variants of the present invention are especially suitable for the use in test strips because they exhibit a more homogeneous glycosylation pattern with less molecular weight distribution as well as an improved temperature stability under dry condition in comparison to FAD-GDHs according to the prior art. The term "under dry condition" has been defined above.

Another subject of the present invention is a method of detecting, determining or measuring glucose in a sample using a modified flavin adenine dinucleotide dependent glucose dehydrogenase or functional fragments thereof according to the invention, said detection, determination or measurement comprising contacting a sample with said modified flavin adenine dinucleotide dependent glucose dehydrogenase or functional fragments thereof. A specific embodiment of said method according to the present invention is a method, wherein said detection, determination or measurement of glucose is performed using a sensor or a test strip device.

Also within the scope of the invention is a device for the detection, determination or measurement of glucose in a sample comprising a modified flavin adenine dinucleotide dependent glucose dehydrogenase or functional fragments thereof according to the present invention, in particular together with other reagents required for said detection, determination or measurement of glucose in a sample.

The FAD-GDH variants with improved thermo stability of this invention can also be advantageously used in biosensors (D'Costa, E. J., et al., Biosensors 2 (1986) 71-87; Laurinavicius, V., et al., Analytical Letters 32 (1999) 299-316; Laurinavicius, V., et al., Monatshefte fuer Chemie 130 (1999) 1269-1281; Malinauskas, A. et al., Sensors and Actuators, B: Chemical 100 (2004) 395-402) for online monitoring of glucose in a sample or a reactor. For this purpose, the FAD-GDH variants can, for example, be used to coat an oxygen-insensitive glassy electrode with an osmium complex containing a redox conductive epoxy network (Ye *et al.,* 1993 supra) for more accurate determination of the glucose concentration.

In view of the stated above, further specific aspects of the invention are as follows:

In a first aspect the present invention relates to a modified flavin adenine dinucleotide dependent glucose dehydrogenase that is glycosylated, selected from the group consisting of a

a) modified flavin adenine dinucleotide dependent glucose dehydrogenase having SEQ ID No: 2 that is glycosylated, but wherein at least one of the asparagine residues selected from the group consisting of N2; N168 and N346 according to the mature *Aspergillus oryzae* FAD-GDH wild-type sequence SEQ ID No: 2 (Wild type) has been substituted by one or more amino acids not suitable for glycosylation and thereby eliminating or inactivating, respectively, a potential glycosylation site at this position and

b) a modified flavin adenine dinucleotide dependent glucose dehydrogenase that is glycosylated that exhibits 80% amino acid sequence identity or more to a modified flavin adenine dinucleotide dependent glucose dehydrogenase consisting of SEQ ID No: 2, but wherein at least one of the asparagine residues selected from the group consisting of N2; N168 and N346 according to the mature *Aspergillus oryzae* FAD-GDH wild-type sequence according to SEQ ID No: 2 has been substituted by one or more amino acids not suitable for glycosylation and thereby eliminating or inactivating, respectively, a potential glycosylation site at this position and wherein said asparagine residue has been substituted by S, P, SP or D, and

c) an active fragment of a modified flavin adenine dinucleotide dependent glucose dehydrogenase according to (a) or (b) provided that in the FAD-GDH according to (b) or the fragment according to (c) said substitution(s) eliminating or inactivating said potential glycosylation site(s) is/are preserved when compared to the modified flavin adenine dinucleotide dependent glucose dehydrogenase according to (a) and provided that the flavin adenine dinucleotide dependent glucose dehydrogenase according to (b) or the fragment according to (c) exhibits at least 80% of the enzyme activity of the FAD-GDH according (a) and exhibits at least 80% of the temperature stability under dry conditions of the FAD-GDH according to (a), wherein the expression "exhibits temperature stability under dry conditions" means residual activity of the lyophilized modified flavin adenine dinucleotide dependent glucose dehydrogenase (FAD-GDH) itself and when comprised in a lyophilized composition, calculated and compared to the unstressed lyophilizate after lyophilization and incubation of the lyophilized enzyme at 80°C for 8 days over molecular sieve (3A, MS551, Grace).

In a second aspect the present invention relates to a modified flavin adenine dinucleotide dependent glucose dehydrogenase or an active fragment thereof according to the first aspect, wherein said modified flavin adenine dinucleotide dependent glucose dehydrogenase exhibits an improved temperature stability under dry conditions in comparison to glycosylated FAD-GDH according to SEQ ID No: 1, wherein said FAD-GDH according to SEQ ID No: 1 is obtainable by expression in *Aspergillus oryzae.*

In a third aspect the present invention relates to a modified flavin adenine dinucleotide dependent glucose dehydrogenase or an active fragment thereof according to the first or the second aspect, wherein said modified flavin adenine dinucleotide dependent glucose dehydrogenase or an active fragment thereof exhibits a degree of glycosylation that is < 50 %, and/ or the ratio of Mw/Mn is < 1.02.

In a fourth aspect the present invention relates to a modified flavin adenine dinucleotide dependent glucose dehydrogenase or an active fragment thereof according to any of the first to third aspects, wherein only one of the asparagine residues selected from the group consisting of N2; N168 and N346 has been substituted by one or more amino acids not suitable for glycosylation and thereby eliminating or inactivating, respectively, a potential glycosylation site at this position.

In a fifth aspect the present invention relates to a modified flavin adenine dinucleotide dependent glucose dehydrogenase or an active fragment thereof according to any of the first to fourth aspects, wherein said modified flavin adenine dinucleotide dependent dehydrogenase or an active fragment thereof derived from *Aspergillus oryzae* FAD-GDH wild-type sequence SEQ ID No: 2 (Wild type) has one or more of the following substitutions N2S, N168P, N168SP, and N346D.

In a sixth aspect the present invention relates to a modified flavin adenine dinucleotide dependent glucose dehydrogenase or an active fragment thereof according to any of the first to fifth aspects, wherein said modified flavin adenine dinucleotide dependent dehydrogenase or an active fragment thereof derived from *Aspergillus oryzae* FAD-GDH wild-type sequence SEQ ID No: 2 (Wild type) has the following substitution N2S (SEQ ID No: 3).

In a seventh aspect the present invention relates to a composition comprising a modified flavin adenine dinucleotide dependent glucose dehydrogenase or an active fragment thereof according to any of the first to sixth aspects, wherein said modified flavin adenine dinucleotide dependent glucose dehydrogenase or an active fragment thereof exhibits a degree of glycosylation that is < 50 %, and/ or the ratio of Mw/Mn is < 1.02.

In an eighth aspect the present invention relates to an isolated polynucleotide encoding a modified flavin adenine dinucleotide dependent glucose dehydrogenase or an active fragment thereof according to any of the first to sixth aspects with the proviso that said isolated polynucleotide is not encoding for a modified flavin adenine dinucleotide dependent glucose dehydrogenase or an active fragment thereof derived from *Aspergillus oryzae* FAD-GDH wild-type sequence SEQ ID No: 2 (Wild type) that has a single substitution selected from the group consisting of N168K, N168P, N168Y, or N168W.

In a ninth aspect the present invention relates to an expression vector containing the isolated polynucleotide according to the eighth aspect.

In a tenth aspect the present invention relates to a host cell comprising the expression vector according to the ninth aspect, including an isolated polynucleotide that is encoding for a modified flavin adenine dinucleotide dependent glucose dehydrogenase or an active fragment thereof derived from *Aspergillus oryzae* FAD-GDH wild-type sequence SEQ ID No: 2 (Wild type) that has a single substitution selected from the group consisting of N168K, N168P, N168Y, or N168W, with the proviso that said host cell is capable of glycosylation, particularly for *N*-linked glycosylation by having endogenous glycosylating enzymes, and said host cell is not an *E.coli* strain.

In an eleventh aspect the present invention relates to a process for producing a modified flavin adenine dinucleotide dependent glucose dehydrogenase or an active fragment thereof, the process comprising culturing the transformant according to the tenth aspect.

In a twelfth aspect the present invention relates to a modified flavin adenine dinucleotide dependent glucose dehydrogenase or an active fragment thereof obtainable by the process of the eleventh aspect.

In a thirteenth aspect the present invention relates to a method of detecting, determining or measuring glucose in an *ex vivo* sample using a modified flavin adenine dinucleotide dependent glucose dehydrogenase or an active fragment thereof according to any of the first to sixth aspects, or the twelfth aspect or a composition according to the seventh aspect, said detection, determination or measurement comprising contacting an *ex vivo* sample with said modified flavin adenine dinucleotide dependent glucose dehydrogenase or an active fragment thereof or composition, respectively.

In a fourteenth aspect the present invention relates to the method of the thirteenth aspect, wherein said detection, determination or measurement of glucose is performed using a sensor or a test strip device.

In a fifteenth aspect the present invention relates to a device for the detection, determination or measurement of glucose in an *ex vivo* sample comprising a modified flavin adenine dinucleotide dependent glucose dehydrogenase or an active fragment thereof according to any of the first to sixth aspects, or the twelfth aspect or a composition according to the seventh aspect.

In the following examples, all reagents, restriction enzymes, and other materials were obtained from Roche Diagnostics Germany, unless other commercial sources are specified, and used according to the instructions given by the suppliers. Operations and methods employed for the purification, characterization and cloning of DNA are well known in the art (Ausubel, F., et al., in "Current protocols in molecular biology" (1994), Wiley) and can be adapted as required by the skilled artisan.

The following Examples further illustrate the present invention. These examples are not intended to limit the scope of the present invention, but provide further understanding of the present invention.

**Examples**

**Example 1**

**Expression of the modified FAD-GDHs**

In order to generate suitable vectors for the recombinant expression of FAD-GDH variants in *Pichia pastoris,* the synthetic FAD-GDH wild type gene (SEQ ID No: 7) ligated into a derivative of plasmid pBluescript SK (Stratagene, La Jolla) was used. In a first step an intrinsic recognition site for restriction endonuclease *Xho*I was eliminated by a silent mutation by use of the Quick Change II site-directed mutagenesis kit (Stratagene, La Jolla) and mutagenic primers (SEQ ID No: 8, SEQ ID No: 9) resulting in SEQ ID No: 10. This construct was used as a template to add flanking sequences including an *Xho*I site at the 5'- and an *Age*I site at the 3'-end by PCR amplification using PCR primers with SEQ ID No: 11 and SEQ ID No: 12. After amplification the PCR product was hydrolysed with restriction endonucleases *Xho*I and *Age*I (New England Biolabs) and ligated into the *Xho*I/*Age*I hydrolysed expression vector pPICZαA (Invitrogen) resulting in a fusion gene (SEQ ID No: 13) coding for the α-factor signal sequence, a proteolytic cleavage site (KEX2) and the mature FAD-GDH. In order to introduce the single amino acid substitution pPICZαA carrying SEQ ID No: 13 was used as a template for site-directed mutagenesis. For the individual substitutions mutagenic primer pairs were used as seen in Table 1 together with the Quick Change II site-directed mutagenesis Kit (Stratagene, La Jolla) according to the manufacturer instructions.

**Table 1: Amino acid substitutions, mutagenic primer pairs and resulting sequences**

| AA substitution | mutagenic primer pairs | resulting DNA sequence |
|---|---|---|
| N2S | SEQ ID No: 14<br>SEQ ID No: 15 | SEQ ID No: 16 |
| N168P | SEQ ID No: 17<br>SEQ ID No: 18 | SEQ ID No: 19 |
| N168SP | SEQ ID No: 20<br>SEQ ID No: 21 | SEQ ID No: 22 |
| N346D | SEQ ID No: 23<br>SEQ ID No: 24 | SEQ ID No: 25 |

To generate the corresponding recombinant expression strains, electrocompetent cells of *Pichia pastoris* strain X33 (Invitrogen) were transfected by electroporation with 5 - 10 μg of linearized pPICZαA carrying the DNA encoding the corresponding FAD-GDH variants (SEQ ID No: 16, No: 19, No: 22 and No: 25, respectively). All experimental steps were performed according to the manufacturer's instructions. Transfected cells were plated on YPD agar plates (1% yeast extract, 2% peptone, 2% dextrose (glucose)) containing 100 μg/ml, 250 μg/ml or 500 μg/ml Zeocin as a selection marker and incubated at 28°C for 2 - 3 days.

In order to test productivity of the transfected *P. pastoris* clones, a number of single colonies were picked from the selection plates and inoculated in 4 ml of BMMY medium (1% yeast extract, 2% peptone, 100 mM potassium phosphate, pH 6.0, 1.34% yeast nitrogen base (YNB, Invitrogen), 0,0004% biotin). Expression of the recombinant genes was induced by adding 0.5% methanol each day. Cultures were inoculated up to 7 days at 200 rpm and 28°C. Cell density was measured spectrophotometrically (O.D.600). FAD-GDH activity in the supernatants was determined in a spectrophotometric enzyme assay.

Finally the best producers were transferred into 10 L fermentations in order to obtain enough material for purification and biochemical characterization of the different FAD-GDH variants.

## Example 2

### Purification of the modified FAD-GDHs

1L blank filtered supernatant from the fermentation was concentrated by ultra filtration/ultra dialysis to 0.05 L and adjusted to pH 7.5 using 20 mM potassium phosphate buffer. Afterwards, the supernatant was adjusted to a 2.5 M concentration of ammonium sulfate by the addition of solid ammonium sulfate. After incubation about 1h at room temperature, the solution was centrifuged und the sediment was discarded. The clear supernatant was applied to a 1000 ml phenyl sepharose column. After washing the column with 3 L of a 20 mM potassium phosphate buffer pH 7.5 and a ammonium sulfate concentration of 2.5 M, the FAD-GDH was eluted by a linear gradient of a 20 mM potassium phosphate buffer pH 7.5 and a ammonium sulfate concentration of 2.5 M after a 20 mM potassium phosphate buffer pH 7.5 (5 L). The fractions containing FAD-GDH were collected, purified and concentrated by ultra filtration/ ultra dialysis to about 0.05 L and concentrated in 20 mM Tris/HCl buffer pH 8.5. The sample was applied to a 500 ml Q-sepharose column, washed with 2.5 L of a 20 mM Tris/ HCl buffer pH 8.5 and eluted by a linear gradient (5 L) of 20 mM Tris/HCl buffer pH 8.5 with 100 mM NaCl. The FAD-GDH containing fractions were collected, purified and concentrated to a protein concentration of about 50 mg/ml in 100 mM PIPES buffer pH 7.1 by ultra filtration/ultra dialysis. The resulting sample was lyophilized.

## Example 3

### Enzyme activity and sugar specificity of modified flavin adenine dinucleotide dependent glucose dehydrogenases or functional fragments thereof

#### i) Determining enzyme activity (1M D-glucose as substrate)

50 mM PIPES buffer solution pH 6.5 (including 0.1 % Triton X-100), 163 mM PMS solution, 6.8 mM 2,6-dichlorophenol indophenol (DCPIP) solution, 1 M D-glucose solution, 15.6 ml of the aforementioned PIPES buffer, 0.2 ml of DCPIP solution and 4 ml of D-glucose were mixed to make the reaction agent.

#### ii) Determining sugar specificity (1M maltose or 1M xylose as substrate)

50 mM PIPES buffer solution pH 6.5 (including 0.1 % Triton X-100), 163 mM PMS solution, 6.8 mM 2,6-dichlorophenol indophenol (DCPIP) solution, 1 M D-maltose or D-xylose solution, 15.6 ml of the aforementioned PIPES buffer, 0.2 ml of DCPIP solution and 4 ml of D-maltose or D-xylose solution were mixed to make the reaction agent.

### Measurement Conditions for enzyme activity and sugar specificity

2.9 ml of the respective reaction reagent was pre-heated for 5 minutes at 37°C. 0.1 ml FAD-GDH solution was added and slowly mixed. A spectrometer was calibrated for 5 minutes at 37°C at 600 nm using water as a reference. The absorbance change per minute ($\Delta$ OD TEST) was determined from the linear portion. As blank test, the absorbance change per minute ($\Delta$ OD BLANK) was determined in the same manner as above except that a solvent of the FAD-GDH solution was added to the reagent in place of the FAD-GDH solution. From the values thus obtained, the FAD-GDH activity was calculated by the following equation. In the present invention, one unit (U) of the FAD-GDH activity was defined as the amount of enzyme that reduces 1 $\mu$mol of DCPIP per minute in the presence of

    i) 200 mM D-glucose for determining enzyme activity
    ii) 200 mM D-maltose or D-xylose for determining sugar specificity.

$$\text{Activity (U/ml)} = \{-(\Delta \text{ OD}_{TEST} - \Delta \text{ OD}_{BLANK}) \times 3.0 \times \text{dilution ratio}\}/\{16.3 \times 0.1 \times 1.0\}$$

In the equation, 3.0 is the amount (ml) of respective reaction reagent + enzyme solution, 16.3 is the millimolar molecular absorption coefficient ($cm^2$/micromole) under the conditions for measuring activities of the present invention, 0.1 is the amount of enzyme solution (ml) and 1.0 is the optical light path (cm) of the cell.

## Example 4

**Molecular weight distribution of modified flavin adenine dinucleotide dependent glucose dehydrogenase by SEC-RALS**

10 $\mu$l of a protein solution having a concentration of about 10 mg/ml in 50 mM potassiumphosphate buffer pH 6.9 with 300 mM NaCl were applied to a G3000SWXL TSKgel column (30 cm, Tosoh Biosep). The flow rate of the HPLC pump was 0.7 ml/min. For calibration of the Viscotek Triple detector, a freshly prepared solution of bovine serum albumin (Albumin RPLA4, Art.-Nr. 11 726 544, Roche Diagnostics GmbH) was used. A dn/dc value of 0.185 was used for the evaluation of all samples. The evaluation was carried out using the software OmniSEC 4.7.0 (Malvern Instruments). The Weight Average Molecular Weight ($M_w$) and the Number Average Molecular Weight ($M_n$) was calculated by means of the software from the raw data of the Viscotek Triple Detektors (refraction index (RI) and Right Angle Light Scattering (RALS)). The ratio of $M_w/M_n$ is the polydispersity and gives the size distribution of the protein. Monodisperse proteins have a $M_w/M_n$ value of 1.

Haney, Max: Basics of GPC/SEC with threefold detection, LaborPraxis 28, 50-53 (2004)

Wanda K. Hartmann et al.; Characterization and analysis of thermal denaturation of antibodies by size exclusion high-performance liquid chromatography with quadruple detection, Analytical Biochemistry 325, 227-239 (2004)

Heinzmann, Gerhard; Tartsch, Bernd GPC/SEC - three eyes can see more GIT Spezial Separation 27, 21-24 (2007)

## Example 5

**FAD-GDH activity assay with a C-nitrosoaniline mediator**

**Solution 1 (S1)**

25 mM N,N-bis-(hydroxyethyl)-3-methoxy-nitrosoaniline hydrochloride, (CAS 733686-00-5) with 5%(w/v) PVP (Polyvinylpyrrolidone USP K25, FLUKA #81399) in 100 mM Pipes buffer pH 7.1.

**Solution 2 (S2)**

Saturated ~15% (w/v) 2,18 Phosphormolybdic acid, sodium salt (($Na_6[P_2Mo_{18}O_{62}]$ *24 $H_2O$) CAS 50811-90-0, Honeywell Specialty Chemicals, Article No. 04137) in water.

**Solution 3 (S3)**

1 M Glucose in water.

**Enzyme solution**

Dissolve 10 mg/ml lyophilized enzyme in 100 mM Pipes buffer pH 7.1.
Dilute this about 1:100 in 100 mM Pipes buffer pH 7.1 to get a rate of 0.02-0.05 $\Delta$ E/min.

**Measurement procedure**

**Table 2**

| | |
|---|---|
| S1 | 1000 $\mu$l |
| S2 | 50 $\mu$l |
| S3 | 33 $\mu$l |
| Enzyme solution | 50 $\mu$l |

Measurement of absorption at 724 nm at 25°C for 20 min.

$$\varepsilon_{724\,nm} = 27.5\,[\mathrm{mmol}^{-1} * 1 * \mathrm{cm}^{-1}]$$

**K$_M$-Values for Glucose**

The Glucose concentration in the reaction mixture was varied in the range of 0.1 - 170 mM by changing the Glucose concentration in S3.

For the calculation of the K$_M$ values the measured FAD-GDH activities were fitted to the Michaelis-Menten equation:

$$V = \frac{V_{max} * c}{K_M + c}$$

L. Michaelis, M.L. Menten: Die Kinetik der Invertinwirkung Biochem. Z. 49, 333 - 369 (1913)

v = measured FAD-GDH activities
V$_{Max}$ = maximal FAD-GDH activity
K$_M$ = Michaelis-Menten constant in mM
c = Glucose concentration in mM

## Example 6

**Temperature stability in liquid**

The 10 mg lyophilized enzyme was dissolved per ml 100 mM Pipes buffer pH 7.1. Aliquotes of 1 ml of this solution were stored in closed plastic vials and incubated in temperature controlled water bathes for up to 12 days. The enzyme activities were measured according to Example 3.

## Example 7

**Temperature stability under dry conditions**

The unladed weight of the glass vessel was determined by means of an analytical balance. 10 mg of the lyophilized enzyme sample was weighed. All vessels were closed with plugs in a way that a controlled gas exchange of inner space and the environment was ensured but the sample was prohibited from leaving the vessel. The sample was exposed to 80°C for 8 days in a desiccator in the presence of a drying agent (molecular sieve 3A, MS 551, Grace). After this period, the sample was cooled to room temperature, further allowing the gas exchange with the environment. Afterwards, the vessels were completely closed and the respective weights were determined. In dependence of the original quantity of enzyme, the sample was diluted with ultrapure water to a final concentration of 10 mg/ml completely dissolved by gentle vortexing. The sample was stored for reconstitution for exactly one hour at room temperature and afterwards cooled with ice. Based on this stock solution, dilution took place in ice-cold working buffer, followed by the determination of activity.

**Results**

**Table 3: Exemplary molecular weight distribution SEQ ID No.1 vs. SEQ ID No.3**

| Sample Id | M$_w$ | M$_n$ | M$_w$/M$_n$ |
|---|---|---|---|
| FAD-GDH (SEQ ID No: 1) from *Aspergillus* | 103 876 | 99 901 | 1.040 |
| FAD-GDH (SEQ ID No: 3) variant 1; N2S | 76 333 | 76 147 | 1.002 |

**Molecular weight distribution of modified flavin adenine dinucleotide dependent glucose dehydrogenase by SEC-RALS according to Example 4**

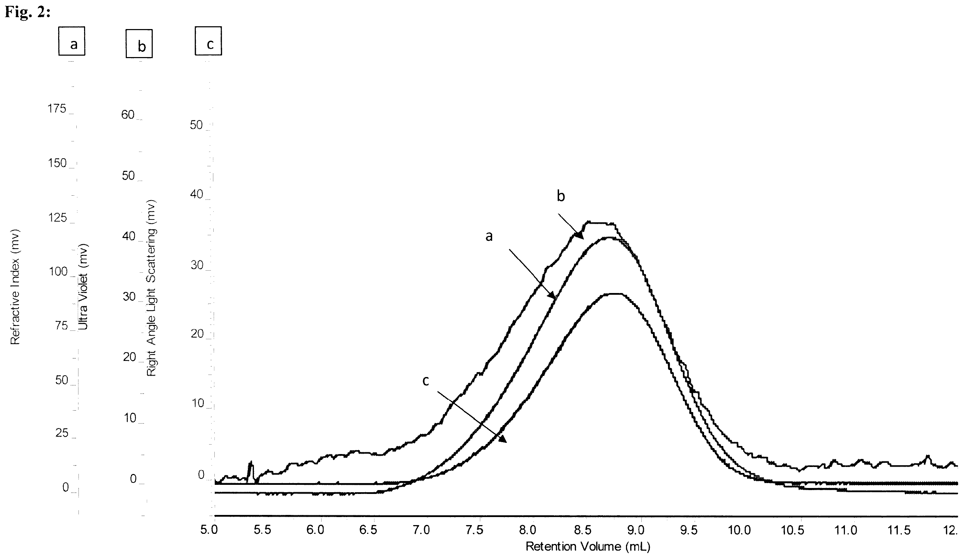

Graph: FAD-GDH (SEQ ID No: 1) from *Aspergillus* see Figure 2.

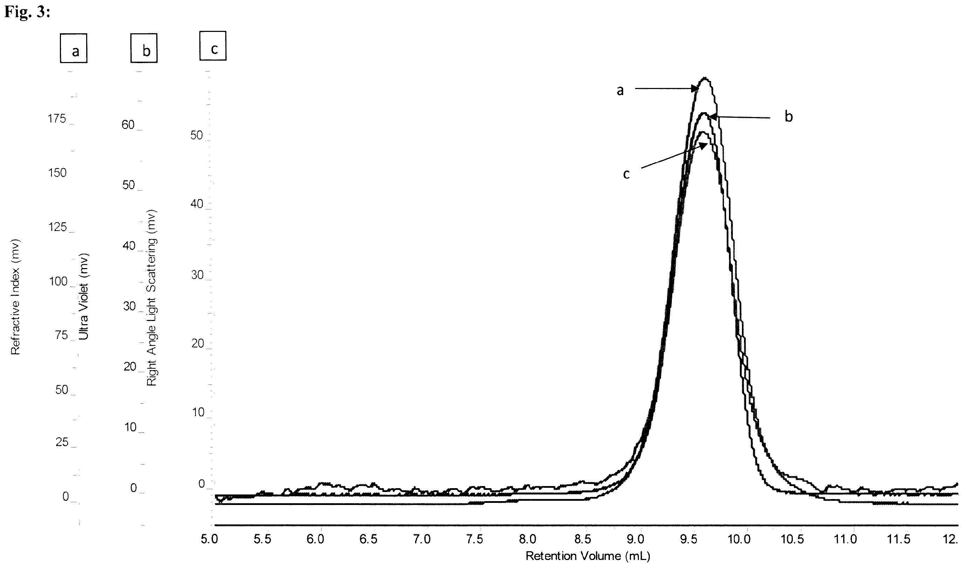

Graph: FAD-GDH (SEQ ID No: 3) variant 1; N2S see Figure 3.

**Temperature stability under dry conditions according to Example 7**

| | |
|---|---|
| Reference: | FAD-GDH according to SEQ ID No: 1: 67 +/- 5% |
| N2S modification (variant 1): | FAD-GDH according to SEQ ID No: 3: 79 +/- 5% |

**Sequence Listing**

SEQ ID No: 1

```
KNTTTYDYIVVGGGTSGLVVANRLSENPDVSVLLLEAGASVFNNPDVTNANGYGLAFGSAID
WQYQSINQSYAGGKQQVLRAGKALGGTSTINGMAYTRAEDVQIDVWQKLGNEGWTWKDLLPY
YLKSENLTAPTSSQVAAGAAYNPAVNGKEGPLKVGWSRSLASGNLSVALNRTFQAAGVPWVE
DVNGGKMRGFNIYPSTLDVDLNVREDAARAYYFPYDDRKNLHLLENTTANRLFWKNGSAEEA
IADGVEITSADGKVTRVHAKKEVIISAGALRSPLILELSGVGNPTILKKNNITPRVDLPTVG
ENLQDQFNNGMAGEGYGVLAGASTVTYPSISDVFGNETDSIVASLRSQLSDYAAATVKVSNG
HMKQEDLERLYQLQFDLIVKDKVPIAEILFHPGGGNAVSSEFWGLLPFARGNIHISSNDPTA
PAAINPNYFMFEWDGKSQAGIAKYIRKILRSAPLNKLIAKETKPGLSEIPATAADEKWVEWL
KANYRSNFHPVGTAAMMPRSIGGVVDNRLRVYGTSNVRVVDASVLPFQVCGHLCSTLYAVAE
RASDLIKEDAKSA
```

SEQ ID No: 2 (Wild type) FAD-GDH wild-type sequence

KNTTTYDYIVVGGGTSGLVVANRLSENPDVSVLLLEAGASVFNNPDVTNANGYGLAFGSAID
WQYQSINQSYAGGKQQVLRAGKALGGTSTINGMAYTRAEDVQIDVWQKLGNEGWTWKDLLPY
YLKSENLTAPTSSQVAAGAAYNPAVNGKEGPLKVGWSGSLASGNLSVALNRTFQAAGVPWVE
DVNGGKMRGFNIYPSTLDVDLNVREDAARAYYFPYDDRKNLHLLENTTANRLFWKNGSAEEA
IADGVEITSADGKVTRVHAKKEVIISAGALRSPLILELSGVGNPTILKKNNITPRVDLPTVG
ENLQDQFNNGMAGEGYGVLAGASTVTYPSISDVFGNETDSIVASLRSQLSDYAAATVKVSNG
HMKQEDLERLYQLQFDLIVKDKVPIAEILFHPGGGNAVSSEFWGLLPFARGNIHISSNDPTA
PAAINPNYFMFEWDGKSQAGIAKYIRKILRSAPLNKLIAKETKPGLSEIPATAADEKWVEWL
KANYRSNFHPVGTAAMMPRSIGGVVDNRLRVYGTSNVRVVDASVLPFQVCGHLVSTLYAVAE
RASDLIKEDAKSA

SEQ ID NO: 3 (N2S)

KSTTTYDYIVVGGGTSGLVVANRLSENPDVSVLLLEAGASVFNNPDVTNANGYGLAFGSAID
WQYQSINQSYAGGKQQVLRAGKALGGTSTINGMAYTRAEDVQIDVWQKLGNEGWTWKDLLPY
YLKSENLTAPTSSQVAAGAAYNPAVNGKEGPLKVGWSGSLASGNLSVALNRTFQAAGVPWVE
DVNGGKMRGFNIYPSTLDVDLNVREDAARAYYFPYDDRKNLHLLENTTANRLFWKNGSAEEA
IADGVEITSADGKVTRVHAKKEVIISAGALRSPLILELSGVGNPTILKKNNITPRVDLPTVG
ENLQDQFNNGMAGEGYGVLAGASTVTYPSISDVFGNETDSIVASLRSQLSDYAAATVKVSNG
HMKQEDLERLYQLQFDLIVKDKVPIAEILFHPGGGNAVSSEFWGLLPFARGNIHISSNDPTA
PAAINPNYFMFEWDGKSQAGIAKYIRKILRSAPLNKLIAKETKPGLSEIPATAADEKWVEWL

KANYRSNFHPVGTAAMMPRSIGGVVDNRLRVYGTSNVRVVDASVLPFQVCGHLVSTLYAVAE
RASDLIKEDAKSA

SEQ ID No: 4 (N168P)

KNTTTYDYIVVGGGTSGLVVANRLSENPDVSVLLLEAGASVFNNPDVTNANGYGLAFGSAID
WQYQSINQSYAGGKQQVLRAGKALGGTSTINGMAYTRAEDVQIDVWQKLGNEGWTWKDLLPY
YLKSENLTAPTSSQVAAGAAYNPAVNGKEGPLKVGWSGSLASGPLSVALNRTFQAAGVPWVE
DVNGGKMRGFNIYPSTLDVDLNVREDAARAYYFPYDDRKNLHLLENTTANRLFWKNGSAEEA
IADGVEITSADGKVTRVHAKKEVIISAGALRSPLILELSGVGNPTILKKNNITPRVDLPTVG
ENLQDQFNNGMAGEGYGVLAGASTVTYPSISDVFGNETDSIVASLRSQLSDYAAATVKVSNG
HMKQEDLERLYQLQFDLIVKDKVPIAEILFHPGGGNAVSSEFWGLLPFARGNIHISSNDPTA
PAAINPNYFMFEWDGKSQAGIAKYIRKILRSAPLNKLIAKETKPGLSEIPATAADEKWVEWL
KANYRSNFHPVGTAAMMPRSIGGVVDNRLRVYGTSNVRVVDASVLPFQVCGHLVSTLYAVAE
RASDLIKEDAKSA

SEQ ID No: 5 (N168SP)

KNTTTYDYIVVGGGTSGLVVANRLSENPDVSVLLLEAGASVFNNPDVTNANGYGLAFGSAID
WQYQSINQSYAGGKQQVLRAGKALGGTSTINGMAYTRAEDVQIDVWQKLGNEGWTWKDLLPY
YLKSENLTAPTSSQVAAGAAYNPAVNGKEGPLKVGWSGSLASGSPLSVALNRTFQAAGVPWV
EDVNGGKMRGFNIYPSTLDVDLNVREDAARAYYFPYDDRKNLHLLENTTANRLFWKNGSAEE
AIADGVEITSADGKVTRVHAKKEVIISAGALRSPLILELSGVGNPTILKKNNITPRVDLPTV
GENLQDQFNNGMAGEGYGVLAGASTVTYPSISDVFGNETDSIVASLRSQLSDYAAATVKVSN
GHMKQEDLERLYQLQFDLIVKDKVPIAEILFHPGGGNAVSSEFWGLLPFARGNIHISSNDPT
APAAINPNYFMFEWDGKSQAGIAKYIRKILRSAPLNKLIAKETKPGLSEIPATAADEKWVEW
LKANYRSNFHPVGTAAMMPRSIGGVVDNRLRVYGTSNVRVVDASVLPFQVCGHLVSTLYAVA
ERASDLIKEDAKSA

SEQ ID No: 6 (N346D)

KNTTTYDYIVVGGGTSGLVVANRLSENPDVSVLLLEAGASVFNNPDVTNANGYGLAFGSAID
WQYQSINQSYAGGKQQVLRAGKALGGTSTINGMAYTRAEDVQIDVWQKLGNEGWTWKDLLPY
YLKSENLTAPTSSQVAAGAAYNPAVNGKEGPLKVGWSGSLASGNLSVALNRTFQAAGVPWVE
DVNGGKMRGFNIYPSTLDVDLNVREDAARAYYFPYDDRKNLHLLENTTANRLFWKNGSAEEA
IADGVEITSADGKVTRVHAKKEVIISAGALRSPLILELSGVGNPTILKKNNITPRVDLPTVG
ENLQDQFNNGMAGEGYGVLAGASTVTYPSISDVFGDETDSIVASLRSQLSDYAAATVKVSNG
HMKQEDLERLYQLQFDLIVKDKVPIAEILFHPGGGNAVSSEFWGLLPFARGNIHISSNDPTA

PAAINPNYFMFEWDGKSQAGIAKYIRKILRSAPLNKLIAKETKPGLSEIPATAADEKWVEWL
KANYRSNFHPVGTAAMMPRSIGGVVDNRLRVYGTSNVRVVDASVLPFQVCGHLVSTLYAVAE
RASDLIKEDAKSA

SEQ ID No: 7 (FAD-GDH wild type)

5'-AAGAACACTACGACATACGACTACATCGTTGTGGGAGGCGGCACAAGTGGTCTTGTGGT
CGCAAATCGCCTTTCTGAGAACCCCGATGTCTCCGTTCTTCTGCTTGAGGCCGGTGCTTCTG
TGTTCAACAACCCGGACGTAACCAACGCTAACGGTTATGGATTGGCCTTTGGCTCGGCCATC
GACTGGCAGTACCAGTCTATTAACCAAAGCTATGCAGGAGGTAAACAGCAAGTTCTGCGTGC
TGGTAAGGCCCTTGGAGGAACCAGTACAATCAATGGAATGGCCTATACCCGCGCAGAGGATG
TCCAGATTGACGTTTGGCAGAAACTTGGAAACGAAGGTTGGACGTGGAAAGATCTCCTACCA
TACTACCTGAAGAGTGAAAACTTGACGGCCCCTACCAGCTCTCAGGTTGCTGCTGGCGCTGC
TTATAACCCTGCCGTGAATGGAAAAGAAGGTCCTCTCAAGGTCGGCTGGTCGGGAAGCCTGG
CCTCCGGTAATCTGTCAGTTGCTCTGAACCGTACGTTCCAAGCCGCTGGTGTTCCATGGGTT
GAGGATGTCAATGGAGGCAAGATGCGTGGCTTCAACATCTACCCATCCACCCTCGACGTTGA
CCTCAATGTCCGCGAAGATGCAGCCCGGGCATACTACTTCCCTTATGATGACAGGAAGAACC
TTCACCTGCTGGAGAACACCACTGCCAACCGCCTTTTCTGGAAGAACGGCTCTGCTGAGGAA
GCTATTGCGGATGGTGTCGAGATCACCTCCGCTGATGGCAAGGTCACTCGTGTGCATGCAAA
GAAAGAGGTCATCATCTCTGCTGGTGCCCTGCGGTCTCCTCTCATTCTCGAGCTTTCAGGAG
TTGGAAACCCAACCATCCTCAAAAAGAACAACATAACCCCACGTGTCGATCTCCCCACCGTT
GGGGAGAACCTCCAAGACCAGTTCAACAACGGCATGGCTGGCGAAGGATACGGCGTCCTTGC
CGGTGCCTCAACCGTGACCTACCCTTCCATCTCCGACGTCTTCGGTAACGAGACTGACTCTA
TCGTTGCATCTCTCCGATCTCAACTCTCCGACTACGCCGCCGCGACCGTCAAGGTCAGCAAC
GGCCACATGAAGCAGGAGGACCTTGAGCGCCTCTACCAGCTCCAATTTGACCTCATCGTCAA
GGACAAGGTCCCTATCGCCGAGATCCTCTTCCACCCCGGTGGTGGAAACGCCGTGTCCTCCG
AATTCTGGGGCTTGCTTCCCTTCGCCCGTGGCAACATCCACATTAGCTCCAATGACCCGACT
GCTCCCGCCGCCATCAACCCTAACTACTTTATGTTCGAATGGGACGGCAAGAGCCAGGCCGG
TATCGCCAAGTACATCAGGAAGATTCTCCGCAGCGCACCATTGAACAAACTTATTGCGAAGG
AAACCAAGCCCGGTCTCTCTGAGATTCCGGCCACTGCTGCGGATGAGAAGTGGGTTGAATGG
CTCAAGGCTAACTATCGTTCCAACTTCCACCCCGTCGGAACTGCTGCCATGATGCCTCGTTC
CATTGGTGGCGTTGTTGATAACCGTCTCCGGGTCTATGGTACCAGCAATGTTCGCGTCGTAG
ATGCGTCTGTCCTGCCCTTCCAGGTTTGCGGCCACTTGGTTAGCACGCTTTATGCCGTTGCC
GAGCGCGCTTCCGACTTGATTAAGGAGGATGCGAAGAGTGCTTAG-3'

SEQ ID No: 8 (forward primer ΔXhoI)
5'-ggtctcctctcattcttgagctttcaggagttgg-3'

SEQ ID No: 9 (reverse primer ΔXhoI))
5'- ccaactcctgaaagctcaagaatgagaggagacc-3'

SEQ ID No: 10 (wild-type FAD-GDH ΔXhoI)

5'-AAGAACACTACGACATACGACTACATCGTTGTGGGAGGCGGCACAAGTGGTCTTGTGGT
CGCAAATCGCCTTTCTGAGAACCCCGATGTCTCCGTTCTTCTGCTTGAGGCCGGTGCTTCTG
TGTTCAACAACCCGGACGTAACCAACGCTAACGGTTATGGATTGGCCTTTGGCTCGGCCATC
GACTGGCAGTACCAGTCTATTAACCAAAGCTATGCAGGAGGTAAACAGCAAGTTCTGCGTGC
TGGTAAGGCCCTTGGAGGAACCAGTACAATCAATGGAATGGCCTATACCCGCGCAGAGGATG
TCCAGATTGACGTTTGGCAGAAACTTGGAAACGAAGGTTGGACGTGGAAAGATCTCCTACCA
TACTACCTGAAGAGTGAAAACTTGACGGCCCCTACCAGCTCTCAGGTTGCTGCTGGCGCTGC
TTATAACCCTGCCGTGAATGGAAAAGAAGGTCCTCTCAAGGTCGGCTGGTCGGGAAGCCTGG
CCTCCGGTAATCTGTCAGTTGCTCTGAACCGTACGTTCCAAGCCGCTGGTGTTCCATGGGTT
GAGGATGTCAATGGAGGCAAGATGCGTGGCTTCAACATCTACCCATCCACCCTCGACGTTGA
CCTCAATGTCCGCGAAGATGCAGCCCGGGCATACTACTTCCCTTATGATGACAGGAAGAACC
TTCACCTGCTGGAGAACACCACTGCCAACCGCCTTTTCTGGAAGAACGGCTCTGCTGAGGAA
GCTATTGCGGATGGTGTCGAGATCACCTCCGCTGATGGCAAGGTCACTCGTGTGCATGCAAA
GAAAGAGGTCATCATCTCTGCTGGTGCCCTGCGGTCTCCTCTCATTCTTGAGCTTTCAGGAG
TTGGAAACCCAACCATCCTCAAAAAGAACAACATAACCCCACGTGTCGATCTCCCCACCGTT
GGGGAGAACCTCCAAGACCAGTTCAACAACGGCATGGCTGGCGAAGGATACGGCGTCCTTGC
CGGTGCCTCAACCGTGACCTACCCTTCCATCTCCGACGTCTTCGGTAACGAGACTGACTCTA
TCGTTGCATCTCTCCGATCTCAACTCTCCGACTACGCCGCCGCGACCGTCAAGGTCAGCAAC
GGCCACATGAAGCAGGAGGACCTTGAGCGCCTCTACCAGCTCCAATTTGACCTCATCGTCAA
GGACAAGGTCCCTATCGCCGAGATCCTCTTCCACCCCGGTGGTGGAAACGCCGTGTCCTCCG
AATTCTGGGGCTTGCTTCCCTTCGCCCGTGGCAACATCCACATTAGCTCCAATGACCCGACT
GCTCCCGCCGCCATCAACCCTAACTACTTTATGTTCGAATGGGACGGCAAGAGCCAGGCCGG
TATCGCCAAGTACATCAGGAAGATTCTCCGCAGCGCACCATTGAACAAACTTATTGCGAAGG
AAACCAAGCCCGGTCTCTCTGAGATTCCGGCCACTGCTGCGGATGAGAAGTGGGTTGAATGG
CTCAAGGCTAACTATCGTTCCAACTTCCACCCCGTCGGAACTGCTGCCATGATGCCTCGTTC
CATTGGTGGCGTTGTTGATAACCGTCTCCGGGTCTATGGTACCAGCAATGTTCGCGTCGTAG
ATGCGTCTGTCCTGCCCTTCCAGGTTTGCGGCCACTTGGTTAGCACGCTTTATGCCGTTGCC
GAGCGCGCTTCCGACTTGATTAAGGAGGATGCGAAGAGTGCTTAG-3'

SEQ ID No: 11 (forward primer)
5'-ATGCCTCGAGAAAAGAGAGGCTGAAGCTAAGAACACTACGACATACGACTACATC-3'

SEQ ID No: 12 (reverse primer)

5'-GCATACCGGTCTTCTCGTAAGTGCCCAACTTGAACTGAGGAACAGTCATGTCTAAGGCT
ACAAACTCATTAAGCACTCTTCGCATCCTCCTTAATC-3'

SEQ ID No: 13 (FAD-GDH wild-type gene + α-factor signal sequence and KEX2 site)

5′-ATGAGATTTCCTTCAATTTTTACTGCTGTTTTATTCGCAGCATCCTCCGCATTAGCTGC
TCCAGTCAACACTACAACAGAAGATGAAACGGCACAATTCCGGCTGAAGCTGTCATCGGTT
ACTCAGATTAGAAGGGGATTTCGATGTTGCTGTTTTGCCATTTTCCAACAGCACAAATAAC
GGGTTATTGTTTATAAATACTACTATTGCCAGCATTGCTGCTAAAGAAGAAGGGGTATCTCT
CGAGAAAAGAAAGAACACTACGACATACGACTACATCGTTGTGGGAGGCGGCACAAGTGGTC
TTGTGGTCGCAAATCGCCTTTCTGAGAACCCCGATGTCTCCGTTCTTCTGCTTGAGGCCGGT
GCTTCTGTGTTCAACAACCCGGACGTAACCAACGCTAACGGTTATGGATTGGCCTTTGGCTC
GGCCATCGACTGGCAGTACCAGTCTATTAACCAAAGCTATGCAGGAGGTAAACAGCAAGTTC
TGCGTGCTGGTAAGGCCCTTGGAGGAACCAGTACAATCAATGGAATGGCCTATACCCGCGCA
GAGGATGTCCAGATTGACGTTTGGCAGAAACTTGGAAACGAAGGTTGGACGTGGAAAGATCT
CCTACCATACTACCTGAAGAGTGAAAACTTGACGGCCCCTACCAGCTCTCAGGTTGCTGCTG
GCGCTGCTTATAACCCTGCCGTGAATGGAAAGAAGGTCCTCTCAAGGTCGGCTGGTCGGGA
AGCCTGGCCTCCGGTAATCTGTCAGTTGCTCTGAACCGTACGTTCCAAGCCGCTGGTGTTCC
ATGGGTTGAGGATGTCAATGGAGGCAAGATGCGTGGCTTCAACATCTACCCATCCACCCTCG
ACGTTGACCTCAATGTCCGCGAAGATGCAGCCCGGGCATACTACTTCCCTTATGATGACAGG
AAGAACCTTCACCTGCTGGAGAACACCACTGCCAACCGCCTTTTCTGGAAGAACGGCTCTGC
TGAGGAAGCTATTGCGGATGGTGTCGAGATCACCTCCGCTGATGGCAAGGTCACTCGTGTGC
ATGCAAAGAAAGAGGTCATCATCTCTGCTGGTGCCCTGCGGTCTCCTCTCATTCTTGAGCTT
TCAGGAGTTGGAAACCCAACCATCCTCAAAAAGAACAACATAACCCCACGTGTCGATCTCCC
CACCGTTGGGGAGAACCTCCAAGACCAGTTCAACAACGGCATGGCTGGCGAAGGATACGGCG
TCCTTGCCGGTGCCTCAACCGTGACCTACCCTTCCATCTCCGACGTCTTCGGTAACGAGACT
GACTCTATCGTTGCATCTCTCCGATCTCAACTCTCCGACTACGCCGCCGCGACCGTCAAGGT
CAGCAACGGCCACATGAAGCAGGAGGACCTTGAGCGCCTCTACCAGCTCCAATTTGACCTCA
TCGTCAAGGACAAGGTCCCTATCGCCGAGATCCTCTTCCACCCCGGTGGTGGAAACGCCGTG
TCCTCCGAATTCTGGGGCTTGCTTCCCTTCGCCCGTGGCAACATCCACATTAGCTCCAATGA
CCCGACTGCTCCCGCCGCCATCAACCCTAACTACTTTATGTTCGAATGGGACGGCAAGAGCC

AGGCCGGTATCGCCAAGTACATCAGGAAGATTCTCCGCAGCGCACCATTGAACAAACTTATT
GCGAAGGAAACCAAGCCCGGTCTCTCTGAGATTCCGGCCACTGCTGCGGATGAGAAGTGGGT
TGAATGGCTCAAGGCTAACTATCGTTCCAACTTCCACCCCGTCGGAACTGCTGCCATGATGC
CTCGTTCCATTGGTGGCGTTGTTGATAACCGTCTCCGGGTCTATGGTACCAGCAATGTTCGC
GTCGTAGATGCGTCTGTCCTGCCCTTCCAGGTTTGCGGCCACTTGGTTAGCACGCTTTATGC
CGTTGCCGAGCGCGCTTCCGACTTGATTAAGGAGGATGCGAAGAGTGCTTAA-3′

SEQ ID No: 14
5'-CTCTCGAGAAAAGAAAGTCCACTACGACATACGAC-3'

SEQ ID No: 15
5'-GTCGTATGTCGTAGTGGACTTTCTTTTCTCGAGAG-3'

SEQ ID No: 16

5'-ATGAGATTTCCTTCAATTTTTACTGCTGTTTTATTCGCAGCATCCTCCGCATTAGCTGC
TCCAGTCAACACTACAACAGAAGATGAAACGGCACAAATTCCGGCTGAAGCTGTCATCGGTT
ACTCAGATTTAGAAGGGGATTTCGATGTTGCTGTTTTGCCATTTTCCAACAGCACAAATAAC
GGGTTATTGTTTATAAATACTACTATTGCCAGCATTGCTGCTAAAGAAGAAGGGGTATCTCT
CGAGAAAAGAAAGTCCACTACGACATACGACTACATCGTTGTGGGAGGCGGCACAAGTGGTC
TTGTGGTCGCAAATCGCCTTTCTGAGAACCCCGATGTCTCCGTTCTTCTGCTTGAGGCCGGT
GCTTCTGTGTTCAACAACCCGGACGTAACCAACGCTAACGGTTATGGATTGGCCTTTGGCTC
GGCCATCGACTGGCAGTACCAGTCTATTAACCAAAGCTATGCAGGAGGTAAACAGCAAGTTC
TGCGTGCTGGTAAGGCCCTTGGAGGAACCAGTACAATCAATGGAATGGCCTATACCCGCGCA
GAGGATGTCCAGATTGACGTTTGGCAGAAACTTGGAAACGAAGGTTGGACGTGGAAAGATCT
CCTACCATACTACCTGAAGAGTGAAAACTTGACGGCCCCTACCAGCTCTCAGGTTGCTGCTG
GCGCTGCTTATAACCCTGCCGTGAATGGAAAAGAAGGTCCTCTCAAGGTCGGCTGGTCGGGA
AGCCTGGCCTCCGGTAATCTGTCAGTTGCTCTGAACCGTACGTTCCAAGCCGCTGGTGTTCC
ATGGGTTGAGGATGTCAATGGAGGCAAGATGCGTGGCTTCAACATCTACCCATCCACCCTCG
ACGTTGACCTCAATGTCCGCGAAGATGCAGCCCGGGCATACTACTTCCCTTATGATGACAGG
AAGAACCTTCACCTGCTGGAGAACACCACTGCCAACCGCCTTTTCTGGAAGAACGGCTCTGC
TGAGGAAGCTATTGCGGATGGTGTCGAGATCACCTCCGCTGATGGCAAGGTCACTCGTGTGC
ATGCAAAGAAAGAGGTCATCATCTCTGCTGGTGCCCTGCGGTCTCCTCTCATTCTTGAGCTT
TCAGGAGTTGGAAACCCAACCATCCTCAAAAAGAACAACATAACCCCACGTGTCGATCTCCC
CACCGTTGGGGAGAACCTCCAAGACCAGTTCAACAACGGCATGGCTGGCGAAGGATACGGCG
TCCTTGCCGGTGCCTCAACCGTGACCTACCCTTCCATCTCCGACGTCTTCGGTAACGAGACT

```
GACTCTATCGTTGCATCTCTCCGATCTCAACTCTCCGACTACGCCGCCGCGACCGTCAAGGT
CAGCAACGGCCACATGAAGCAGGAGGACCTTGAGCGCCTCTACCAGCTCCAATTTGACCTCA
TCGTCAAGGACAAGGTCCCTATCGCCGAGATCCTCTTCCACCCCGGTGGTGGAAACGCCGTG
TCCTCCGAATTCTGGGGCTTGCTTCCCTTCGCCCGTGGCAACATCCACATTAGCTCCAATGA
CCCGACTGCTCCCGCCGCCATCAACCCTAACTACTTTATGTTCGAATGGGACGGCAAGAGCC
AGGCCGGTATCGCCAAGTACATCAGGAAGATTCTCCGCAGCGCACCATTGAACAAACTTATT
GCGAAGGAAACCAAGCCCGGTCTCTCTGAGATTCCGGCCACTGCTGCGGATGAGAAGTGGGT
TGAATGGCTCAAGGCTAACTATCGTTCCAACTTCCACCCCGTCGGAACTGCTGCCATGATGC
CTCGTTCCATTGGTGGCGTTGTTGATAACCGTCTCCGGGTCTATGGTACCAGCAATGTTCGC
GTCGTAGATGCGTCTGTCCTGCCCTTCCAGGTTTGCGGCCACTTGGTTAGCACGCTTTATGC
CGTTGCCGAGCGCGCTTCCGACTTGATTAAGGAGGATGCGAAGAGTGCTTAA-3'
```

SEQ ID No: 17
5'-GCCTGGCCTCCGGTCCTCTGTCAGTTGCTC-3'

SEQ ID No: 18
5'-GAGCAACTGACAGAGGACCGGAGGCCAGGC-3'

SEQ ID No: 19

```
5'-ATGAGATTTCCTTCAATTTTTACTGCTGTTTTATTCGCAGCATCCTCCGCATTAGCTGC
TCCAGTCAACACTACAACAGAAGATGAAACGGCACAAATTCCGGCTGAAGCTGTCATCGGTT
ACTCAGATTTAGAAGGGGATTTCGATGTTGCTGTTTTGCCATTTTCCAACAGCACAAATAAC
GGGTTATTGTTTATAAATACTACTATTGCCAGCATTGCTGCTAAAGAAGAAGGGGTATCTCT
CGAGAAAGAAAGAACACTACGACATACGACTACATCGTTGTGGGAGGCGGCACAAGTGGTC
TTGTGGTCGCAAATCGCCTTTCTGAGAACCCCGATGTCTCCGTTCTTCTGCTTGAGGCCGGT
GCTTCTGTGTTCAACAACCCGGACGTAACCAACGCTAACGGTTATGGATTGGCCTTTGGCTC
GGCCATCGACTGGCAGTACCAGTCTATTAACCAAAGCTATGCAGGAGGTAAACAGCAAGTTC
TGCGTGCTGGTAAGGCCCTTGGAGGAACCAGTACAATCAATGGAATGGCCTATACCCGCGCA
GAGGATGTCCAGATTGACGTTTGGCAGAAACTTGGAAACGAAGGTTGGACGTGGAAAGATCT
CCTACCATACTACCTGAAGAGTGAAAACTTGACGGCCCCTACCAGCTCTCAGGTTGCTGCTG
GCGCTGCTTATAACCCTGCCGTGAATGGAAAAGAAGGTCCTCTCAAGGTCGGCTGGTCGGGA
AGCCTGGCCTCCGGTCCTCTGTCAGTTGCTCTGAACCGTACGTTCCAAGCCGCTGGTGTTCC
ATGGGTTGAGGATGTCAATGGAGGCAAGATGCGTGGCTTCAACATCTACCCATCCACCCTCG
ACGTTGACCTCAATGTCCGCGAAGATGCAGCCCGGGCATACTACTTCCCTTATGATGACAGG
AAGAACCTTCACCTGCTGGAGAACACCACTGCCAACCGCCTTTTCTGGAAGAACGGCTCTGC
```

TGAGGAAGCTATTGCGGATGGTGTCGAGATCACCTCCGCTGATGGCAAGGTCACTCGTGTGC
ATGCAAAGAAAGAGGTCATCATCTCTGCTGGTGCCCTGCGGTCTCCTCTCATTCTTGAGCTT
TCAGGAGTTGGAAACCCAACCATCCTCAAAAAGAACAACATAACCCCACGTGTCGATCTCCC
CACCGTTGGGGAGAACCTCCAAGACCAGTTCAACAACGGCATGGCTGGCGAAGGATACGGCG
TCCTTGCCGGTGCCTCAACCGTGACCTACCCTTCCATCTCCGACGTCTTCGGTAACGAGACT
GACTCTATCGTTGCATCTCTCCGATCTCAACTCTCCGACTACGCCGCCGCGACCGTCAAGGT
CAGCAACGGCCACATGAAGCAGGAGGACCTTGAGCGCCTCTACCAGCTCCAATTTGACCTCA
TCGTCAAGGACAAGGTCCCTATCGCCGAGATCCTCTTCCACCCCGGTGGTGGAAACGCCGTG
TCCTCCGAATTCTGGGGCTTGCTTCCCTTCGCCCGTGGCAACATCCACATTAGCTCCAATGA
CCCGACTGCTCCCGCCGCCATCAACCCTAACTACTTTATGTTCGAATGGGACGGCAAGAGCC
AGGCCGGTATCGCCAAGTACATCAGGAAGATTCTCCGCAGCGCACCATTGAACAAACTTATT
GCGAAGGAAACCAAGCCCGGTCTCTCTGAGATTCCGGCCACTGCTGCGGATGAGAAGTGGGT
TGAATGGCTCAAGGCTAACTATCGTTCCAACTTCCACCCCGTCGGAACTGCTGCCATGATGC
CTCGTTCCATTGGTGGCGTTGTTGATAACCGTCTCCGGGTCTATGGTACCAGCAATGTTCGC
GTCGTAGATGCGTCTGTCCTGCCCTTCCAGGTTTGCGGCCACTTGGTTAGCACGCTTTATGC
CGTTGCCGAGCGCGCTTCCGACTTGATTAAGGAGGATGCGAAGAGTGCTTAA–3'

SEQ ID No: 20
5'-GGGAAGCCTGGCCTCCGGTTCTCCTCTGTCAGTTGCTCTGAACCG-3'

SEQ ID No: 21
5'-CGGTTCAGAGCAACTGACAGAGGAGAACCGGAGGCCAGGCTTCCC-3'

SEQ ID No: 22

5'–ATGAGATTTCCTTCAATTTTTACTGCTGTTTTATTCGCAGCATCCTCCGCATTAGCTGC
TCCAGTCAACACTACAACAGAAGATGAAACGGCACAAATTCCGGCTGAAGCTGTCATCGGTT
ACTCAGATTTAGAAGGGGATTTCGATGTTGCTGTTTTGCCATTTTCCAACAGCACAAATAAC
GGGTTATTGTTTATAAATACTACTATTGCCAGCATTGCTGCTAAAGAAGAAGGGGTATCTCT
CGAGAAAGAAAGAACACTACGACATACGACTACATCGTTGTGGGAGGCGGCACAAGTGGTC
TTGTGGTCGCAAATCGCCTTTCTGAGAACCCCGATGTCTCCGTTCTTCTGCTTGAGGCCGGT
GCTTCTGTGTTCAACAACCCGGACGTAACCAACGCTAACGGTTATGGATTGGCCTTTGGCTC
GGCCATCGACTGGCAGTACCAGTCTATTAACCAAAGCTATGCAGGAGGTAAACAGCAAGTTC
TGCGTGCTGGTAAGGCCCTTGGAGGAACCAGTACAATCAATGGAATGGCCTATACCCGCGCA
GAGGATGTCCAGATTGACGTTTGGCAGAAACTTGGAAACGAAGGTTGGACGTGGAAAGATCT
CCTACCATACTACCTGAAGAGTGAAAACTTGACGGCCCCTACCAGCTCTCAGGTTGCTGCTG

GCGCTGCTTATAACCCTGCCGTGAATGGAAAAGAAGGTCCTCTCAAGGTCGGCTGGTCGGGA
AGCCTGGCCTCCGGTTCTCCTCTGTCAGTTGCTCTGAACCGTACGTTCCAAGCCGCTGGTGT
TCCATGGGTTGAGGATGTCAATGGAGGCAAGATGCGTGGCTTCAACATCTACCCATCCACCC
TCGACGTTGACCTCAATGTCCGCGAAGATGCAGCCCGGGCATACTACTTCCCTTATGATGAC
AGGAAGAACCTTCACCTGCTGGAGAACACCACTGCCAACCGCCTTTTCTGGAAGAACGGCTC
TGCTGAGGAAGCTATTGCGGATGGTGTCGAGATCACCTCCGCTGATGGCAAGGTCACTCGTG
TGCATGCAAAGAAAGAGGTCATCATCTCTGCTGGTGCCCTGCGGTCTCCTCTCATTCTTGAG
CTTTCAGGAGTTGGAAACCCAACCATCCTCAAAAAGAACAACATAACCCCACGTGTCGATCT
CCCCACCGTTGGGGAGAACCTCCAAGACCAGTTCAACAACGGCATGGCTGGCGAAGGATACG
GCGTCCTTGCCGGTGCCTCAACCGTGACCTACCCTTCCATCTCCGACGTCTTCGGTAACGAG
ACTGACTCTATCGTTGCATCTCTCCGATCTCAACTCTCCGACTACGCCGCCGCGACCGTCAA
GGTCAGCAACGGCCACATGAAGCAGGAGGACCTTGAGCGCCTCTACCAGCTCCAATTTGACC
TCATCGTCAAGGACAAGGTCCCTATCGCCGAGATCCTCTTCCACCCCGGTGGTGGAAACGCC
GTGTCCTCCGAATTCTGGGGCTTGCTTCCCTTCGCCCGTGGCAACATCCACATTAGCTCCAA
TGACCCGACTGCTCCCGCCGCCATCAACCCTAACTACTTTATGTTCGAATGGGACGGCAAGA
GCCAGGCCGGTATCGCCAAGTACATCAGGAAGATTCTCCGCAGCGCACCATTGAACAAACTT
ATTGCGAAGGAAACCAAGCCCGGTCTCTCTGAGATTCCGGCCACTGCTGCGGATGAGAAGTG
GGTTGAATGGCTCAAGGCTAACTATCGTTCCAACTTCCACCCCGTCGGAACTGCTGCCATGA
TGCCTCGTTCCATTGGTGGCGTTGTTGATAACCGTCTCCGGGTCTATGGTACCAGCAATGTT
CGCGTCGTAGATGCGTCTGTCCTGCCCTTCCAGGTTTGCGGCCACTTGGTTAGCACGCTTTA
TGCCGTTGCCGAGCGCGCTTCCGACTTGATTAAGGAGGATGCGAAGAGTGCTTAA–3'

SEQ ID No: 23
5'-CCGACGTCTTCGGTGACGAGACTGACTCTATCG-3'

SEQ ID No: 24
5'-CGATAGAGTCAGTCTCGTCACCGAAGACGTCGG-3'

SEQ ID No: 25

5'–ATGAGATTTCCTTCAATTTTTACTGCTGTTTTATTCGCAGCATCCTCCGCATTAGCTGC
TCCAGTCAACACTACAACAGAAGATGAAACGGCACAAATTCCGGCTGAAGCTGTCATCGGTT
ACTCAGATTTAGAAGGGGATTTCGATGTTGCTGTTTTGCCATTTTCCAACAGCACAAATAAC
GGGTTATTGTTTATAAATACTACTATTGCCAGCATTGCTGCTAAAGAAGAAGGGGTATCTCT
CGAGAAAAGAAAGAACACTACGACATACGACTACATCGTTGTGGGAGGCGGCACAAGTGGTC
TTGTGGTCGCAAATCGCCTTTCTGAGAACCCCGATGTCTCCGTTCTTCTGCTTGAGGCCGGT

```
GCTTCTGTGTTCAACAACCCGGACGTAACCAACGCTAACGGTTATGGATTGGCCTTTGGCTC
GGCCATCGACTGGCAGTACCAGTCTATTAACCAAAGCTATGCAGGAGGTAAACAGCAAGTTC
TGCGTGCTGGTAAGGCCCTTGGAGGAACCAGTACAATCAATGGAATGGCCTATACCCGCGCA
GAGGATGTCCAGATTGACGTTTGGCAGAAACTTGGAAACGAAGGTTGGACGTGGAAAGATCT
CCTACCATACTACCTGAAGAGTGAAAACTTGACGGCCCCTACCAGCTCTCAGGTTGCTGCTG
GCGCTGCTTATAACCCTGCCGTGAATGGAAAAGAAGGTCCTCTCAAGGTCGGCTGGTCGGGA
AGCCTGGCCTCCGGTAATCTGTCAGTTGCTCTGAACCGTACGTTCCAAGCCGCTGGTGTTCC
ATGGGTTGAGGATGTCAATGGAGGCAAGATGCGTGGCTTCAACATCTACCCATCCACCCTCG
ACGTTGACCTCAATGTCCGCGAAGATGCAGCCCGGGCATACTACTTCCCTTATGATGACAGG
AAGAACCTTCACCTGCTGGAGAACACCACTGCCAACCGCCTTTTCTGGAAGAACGGCTCTGC
TGAGGAAGCTATTGCGGATGGTGTCGAGATCACCTCCGCTGATGGCAAGGTCACTCGTGTGC
ATGCAAAGAAAGAGGTCATCATCTCTGCTGGTGCCCTGCGGTCTCCTCTCATTCTTGAGCTT
TCAGGAGTTGGAAACCCAACCATCCTCAAAAAGAACAACATAACCCCACGTGTCGATCTCCC
CACCGTTGGGGAGAACCTCCAAGACCAGTTCAACAACGGCATGGCTGGCGAAGGATACGGCG
TCCTTGCCGGTGCCTCAACCGTGACCTACCCTTCCATCTCCGACGTCTTCGGTGACGAGACT
GACTCTATCGTTGCATCTCTCCGATCTCAACTCTCCGACTACGCCGCCGCGACCGTCAAGGT
CAGCAACGGCCACATGAAGCAGGAGGACCTTGAGCGCCTCTACCAGCTCCAATTTGACCTCA
TCGTCAAGGACAAGGTCCCTATCGCCGAGATCCTCTTCCACCCCGGTGGTGGAAACGCCGTG
TCCTCCGAATTCTGGGGCTTGCTTCCCTTCGCCCGTGGCAACATCCACATTAGCTCCAATGA
CCCGACTGCTCCCGCCGCCATCAACCCTAACTACTTTATGTTCGAATGGGACGGCAAGAGCC
AGGCCGGTATCGCCAAGTACATCAGGAAGATTCTCCGCAGCGCACCATTGAACAAACTTATT
GCGAAGGAAACCAAGCCCGGTCTCTCTGAGATTCCGGCCACTGCTGCGGATGAGAAGTGGGT
TGAATGGCTCAAGGCTAACTATCGTTCCAACTTCCACCCCGTCGGAACTGCTGCCATGATGC
CTCGTTCCATTGGTGGCGTTGTTGATAACCGTCTCCGGGTCTATGGTACCAGCAATGTTCGC
GTCGTAGATGCGTCTGTCCTGCCCTTCCAGGTTTGCGGCCACTTGGTTAGCACGCTTTATGC
CGTTGCCGAGCGCGCTTCCGACTTGATTAAGGAGGATGCGAAGAGTGCTTAA-3'
```

**Figure description**

<u>Fig. 1:</u>
*Pichia expression* vector pPICZaA:

    PAOXI = AOX1 promoter (initiates transcription of the gene of interest)
    αFactor-ss = gene encoding the α-Factor signal sequence (gene of interest is fused in frame to this sequence; corresponding polypeptide is secreted into the culture medium by *P. pastoris*)
    TAOX1 = AOX1 terminator (stops transcription of the gene of interest)
    Zeo = Zeomycin resistance gene - selection marker

ColE1 = origin of replication (allows cloning in *E. coli*)

Fig. 2:
Molecular weight distribution of FAD-GDH according to SEQ No. 1 by SEC-RALS.

Fig. 3:
Molecular weight distribution of FAD-GDH according to SEQ No. 3 (variant 1; N2S) by SEC-RALS.

The methods and compositions described herein are representative of specific embodiments and are solely exemplary and not intended to be understood as limitations on the scope of the invention. The invention illustratively described herein suitably may be practiced in the absence of any element or elements, or limitation or limitations, which is not specifically disclosed herein as essential.

The methods and processes illustratively described herein suitably may be practiced in differing orders of steps, and that they are not necessarily restricted to the orders of steps indicated herein or in the attached claims. It is also noted that as used herein and in the appended claims, the singular forms "a," "an," and "the" include plural reference, and the plural include singular forms, unless the context clearly dictates otherwise. Under no circumstances the patent may be interpreted to be limited to the specific examples or embodiments or methods specifically disclosed herein. The invention has been described broadly and generically herein. The terms and expressions that have been employed are used as terms of description and not of limitation, and there is no intent in the use of such terms and expressions to exclude any equivalent of the features shown and described or portions thereof, but it is recognized that various modifications are possible within the scope of the invention as claimed. Thus, it will be understood that although the present invention has been specifically disclosed by its corresponding embodiments and optional features, modification and variation of the concepts disclosed herein may be resorted to by those skilled in the art, and that such modifications and variations are considered to be within the scope of this invention as defined by the appended claims.

**Claims**

1. A modified flavin adenine dinucleotide dependent glucose dehydrogenase that is glycosylated, selected from the group consisting of a

   **(a)** modified flavin adenine dinucleotide dependent glucose dehydrogenase having SEQ ID No: 2 that is glycosylated, but wherein at least one of the asparagine residues selected from the group consisting of N2; N168 and N346 according to the mature *Aspergillus oryzae* FAD-GDH wild-type sequence SEQ ID No: 2 (Wild type) has been substituted by one or more amino acids not suitable for glycosylation and thereby eliminating or inactivating, respectively, a potential glycosylation site at this position and
   **(b)** a modified flavin adenine dinucleotide dependent glucose dehydrogenase that is glycosylated that exhibits 80% amino acid sequence identity or more to a modified flavin adenine dinucleotide dependent glucose dehydrogenase consisting of SEQ ID No: 2, but wherein at least one of the asparagine residues selected from the group consisting of N2; N168 and N346 according to the mature *Aspergillus oryzae* FAD-GDH wild-type sequence SEQ ID No: 2 has been substituted by one or more amino acids not suitable for glycosylation and thereby eliminating or inactivating, respectively, a potential glycosylation site at this position and wherein said asparagine residue has been substituted by S, P, SP or D, and
   **(c)** an active fragment of a modified flavin adenine dinucleotide dependent glucose dehydrogenase according to (a) or (b) provided that in the FAD-GDH according to (b) or the fragment according to (c) said substitution(s) eliminating or inactivating said potential glycosylation site(s) is/are preserved when compared to the modified flavin adenine dinucleotide dependent glucose dehydrogenase according to (a) and provided that the flavin adenine dinucleotide dependent glucose dehydrogenase according to (b) or the fragment according to (c) exhibits at least 80% of the enzyme activity of the FAD-GDH according (a) and exhibits at least 80% of the temperature stability under dry conditions of the FAD-GDH according to (a), wherein the expression "exhibits temperature stability under dry conditions" means residual activity of the lyophilized modified flavin adenine dinucleotide dependent glucose dehydrogenase (FAD-GDH) itself and when comprised in a lyophilized composition, calculated and compared to the unstressed lyophilizate after: 1) lyophilization and incubation of the lyophilized enzyme at 80°C for 8 days over molecular sieve (3A, MS551, Grace).

2. A modified flavin adenine dinucleotide dependent glucose dehydrogenase or an active fragment thereof according to claim 1, wherein said modified flavin adenine dinucleotide dependent glucose dehydrogenase exhibits an improved temperature stability under dry conditions in comparison to glycosylated FAD-GDH according to SEQ ID No: 1, wherein said FAD-GDH according to SEQ ID No: 1 is obtainable by expression in *Aspergillus oryzae.*

3.  A modified flavin adenine dinucleotide dependent glucose dehydrogenase or an active fragment thereof according to claim 1 or 2, wherein said modified flavin adenine dinucleotide dependent glucose dehydrogenase or an active fragment thereof exhibits a degree of glycosylation that is < 50 %, and/ or the ratio of Mw/Mn is < 1,02.

4.  A modified flavin adenine dinucleotide dependent glucose dehydrogenase or an active fragment thereof according to any of claims 1 to 3, wherein only one of the asparagine residues selected from the group consisting of N2; N168 and N346 has been substituted by one or more amino acids not suitable for glycosylation and thereby eliminating or inactivating, respectively, a potential glycosylation site at this position.

5.  A modified flavin adenine dinucleotide dependent glucose dehydrogenase or an active fragment thereof according to any of claims 1 to 4, wherein said modified flavin adenine dinucleotide dependent dehydrogenase or an active fragment thereof derived from *Aspergillus oryzae* FAD-GDH wild-type sequence SEQ ID No: 2 (Wild type) has one or more of the following substitutions N2S, N168P, N168SP, and N346D.

6.  A modified flavin adenine dinucleotide dependent glucose dehydrogenase or an active fragment thereof according to any of claims 1 to 5, wherein said modified flavin adenine dinucleotide dependent dehydrogenase or an active fragment thereof derived from *Aspergillus oryzae* FAD-GDH wild-type sequence SEQ ID No: 2 (Wild type) has the following substitution N2S and is a FAD-GDH according to SEQ ID No: 3 or an active fragment thereof (SEQ ID No: 3).

7.  A composition comprising a modified flavin adenine dinucleotide dependent glucose dehydrogenase or an active fragment thereof according to any of claims 1 to 6, wherein said modified flavin adenine dinucleotide dependent glucose dehydrogenase or an active fragment thereof exhibits a degree of glycosylation that is < 50 %, and/ or the ratio of Mw/Mn is < 1,02.

8.  An isolated polynucleotide encoding a modified flavin adenine dinucleotide dependent glucose dehydrogenase or an active fragment thereof according to any of claims 1 to 6 with the proviso that said isolated polynucleotide is not encoding for a modified flavin adenine dinucleotide dependent glucose dehydrogenase or an active fragment thereof derived from *Aspergillus oryzae* FAD-GDH wild-type sequence SEQ ID No: 2 (Wild type) that has a single substitution selected from the group consisting of N168K, N168P, N168Y, or N168W.

9.  An expression vector containing the isolated polynucleotide according to claim 8.

10. A host cell comprising the expression vector according to claim 9, including an isolated polynucleotide that is encoding for a modified flavin adenine dinucleotide dependent glucose dehydrogenase or an active fragment thereof according to any of claims 1 to 6 with the proviso that said isolated polynucleotide is not encoding for a modified flavin adenine dinucleotide dependent glucose dehydrogenase or an active fragment thereof derived from *Aspergillus oryzae* FAD-GDH wild-type sequence SEQ ID No: 2 (Wild type) that has a single substitution selected from the group consisting of N168K, N168P, N168Y, or N168W, with the proviso that said host cell is capable of glycosylation, particularly for *N*-linked glycosylation by having endogenous glycosylating enzymes, and said host cell is not an *E.coli* strain.

11. A process for producing a modified flavin adenine dinucleotide dependent glucose dehydrogenase or an active fragment thereof, the process comprising culturing the transformant according to claim 10.

12. A modified flavin adenine dinucleotide dependent glucose dehydrogenase or an active fragment thereof according to any of claim 1 to 6 obtainable by the process of claim 11 in a host cell capable of glycosylation.

13. A method of detecting, determining or measuring glucose in an *ex vivo* sample using a modified flavin adenine dinucleotide dependent glucose dehydrogenase or an active fragment thereof according to any of claims 1 to 6, or claim 12 or a composition according to claim 7, said detection, determination or measurement comprising contacting an *ex vivo* sample with said modified flavin adenine dinucleotide dependent glucose dehydrogenase or an active fragment thereof or composition, respectively.

14. The method of claim 13, wherein said detection, determination or measurement of glucose is performed using a sensor or a test strip device.

15. A device for the detection, determination or measurement of glucose in an *ex vivo* sample comprising a modified flavin adenine dinucleotide dependent glucose dehydrogenase or an active fragment thereof according to any of claims 1 to 6, or claim 12 or a composition according to claim 7.

**Patentansprüche**

1. Modifizierte Flavinadenindinucleotid-abhängige Glucose-Dehydrogenase, die glykosyliert ist, ausgewählt aus der Gruppe bestehend aus

   (a) einer modifizierten Flavinadenindinucleotid-abhängigen Glucose-Dehydrogenase mit SEQ ID Nr. 2, die glykosyliert ist, aber wobei mindestens einer der Asparaginreste, ausgewählt aus der Gruppe bestehend aus N2; N168 und N346 gemäß der reifen *Aspergillus oryzae*-FAD-GDH-Wildtyp-Sequenz SEQ ID Nr. 2 (Wildtyp), durch eine oder mehrere nicht zur Glykosylierung geeigneten Aminosäuren substituiert wurde, wodurch eine potenzielle Glykosylierungsstelle an dieser Position eliminiert bzw. inaktiviert wird, und
   (b) einer modifizierten Flavinadenindinucleotid-abhängigen Glucose-Dehydrogenase, die glykosyliert ist, die mindestens 80 % Aminosäuresequenzidentität zu einer aus SEQ ID Nr. 2 bestehenden modifizierten Flavinadenindinucleotid-abhängigen Glucose-Dehydrogenase aufweist, aber wobei mindestens einer der Asparaginreste, ausgewählt aus der Gruppe bestehend aus N2; N168 und N346 gemäß der reifen *Aspergillus oryzae*-FAD-GDH-Wildtyp-Sequenz SEQ ID Nr. 2, durch eine oder mehrere nicht zur Glykosylierung geeigneten Aminosäuren substituiert wurde, wodurch eine potenzielle Glykosylierungsstelle an dieser Position eliminiert bzw. inaktiviert wird und wobei der Asparaginrest durch S, P, SP oder D substituiert wurde, und
   (c) einem aktiven Fragment einer modifizierten Flavinadenindinucleotid-abhängigen Glucose-Dehydrogenase gemäß (a) oder (b), vorausgesetzt, dass in der FAD-GDH gemäß (b) oder in dem Fragment gemäß (c) die Substitution(en), die die potenzielle(n) Glykosylierungsstelle(n) eliminieren oder inaktivieren, im Vergleich zur modifizierten Flavinadenindinucleotid-abhängigen Glucose-Dehydrogenase gemäß (a) erhalten wird/werden und vorausgesetzt, dass die Flavinadenindinucleotid-abhängige Glucose-Dehydrogenase gemäß (b) oder das Fragment gemäß (c) mindestens 80 % der Enzymaktivität der FAD-GDH gemäß (a) aufweist und mindestens 80 % der Temperaturstabilität unter trockenen Bedingungen der FAD-GDH gemäß (a) aufweist, wobei der Ausdruck "weist Temperaturstabilität unter trockenen Bedingungen auf" eine Restaktivität der lyophilisierten modifizierten Flavinadenindinucleotid-abhängigen Glucose-Dehydrogenase (FAD-GDH) selbst und, wenn in einer lyophilisierten Zusammensetzung enthalten, berechnet und verglichen mit dem ungestressten Lyophilisat nach:
   1) Lyophilisierung und Inkubation des lyophilisierten Enzyms bei 80°C für 8 Tage über einem Molekularsieb (3A, MS551, Grace) bedeutet.

2. Modifizierte Flavinadenindinucleotid-abhängige Glucose-Dehydrogenase oder ein aktives Fragment davon nach Anspruch 1, wobei die modifizierte Flavinadenindinucleotid-abhängige Glucose-Dehydrogenase unter trocken Bedingungen im Vergleich zur glykosylierten FAD-GDH gemäß SEQ ID Nr. 1 eine verbesserte Temperaturstabilität aufweist, wobei die FAD-GDH gemäß SEQ ID Nr. 1 durch Expression in *Aspergillus oryzae* erhältlich ist.

3. Modifizierte Flavinadenindinucleotid-abhängige Glucose-Dehydrogenase oder ein aktives Fragment davon nach Anspruch 1 oder 2, wobei die modifizierte Flavinadenindinucleotid-abhängige Glucose-Dehydrogenase oder ein aktives Fragment davon einen Glykosylierungsgrad von <50 % aufweist und/oder das Mw/Mn-Verhältnis <1,02 ist.

4. Modifizierte Flavinadenindinucleotid-abhängige Glucose-Dehydrogenase oder ein aktives Fragment davon nach einem der Ansprüche 1 bis 3, wobei nur einer der Asparaginreste, ausgewählt aus der Gruppe bestehend aus N2; N168 und N346, durch eine oder mehrere nicht zur Glykosylierung geeigneten Aminosäuren substituiert wurde, wodurch eine potenzielle Glykosylierungsstelle an dieser Position eliminiert bzw. inaktiviert wird.

5. Modifizierte Flavinadenindinucleotid-abhängige Glucose-Dehydrogenase oder ein aktives Fragment davon nach einem der Ansprüche 1 bis 4, wobei die modifizierte Flavinadenindinucleotid-abhängige Glucose-Dehydrogenase oder ein aktives Fragment davon, das von der *Aspergillus oryzae*-FAD-GDH-Wildtyp-Sequenz SEQ ID Nr. 2 (Wildtyp) abgeleitet ist, eine oder mehrere der folgenden Substitutionen N2S, N168P, N168SP und N346D aufweist.

6. Modifizierte Flavinadenindinucleotid-abhängige Glucose-Dehydrogenase oder ein aktives Fragment davon nach einem der Ansprüche 1 bis 5, wobei die modifizierte Flavinadenindinucleotid-abhängige Glucose-Dehydrogenase oder ein aktives Fragment davon, das von der *Aspergillus oryzae*-FAD-GDH-Wildtyp-Sequenz SEQ ID Nr. 2 (Wildtyp) abgeleitet ist, die folgende Substitution N2S aufweist und eine FAD-GDH gemäß SEQ ID Nr. 3 oder ein aktives Fragment davon (SEQ ID Nr. 3) ist.

7. Zusammensetzung umfassend eine modifizierte Flavinadenindinucleotid-abhängige Glucose-Dehydrogenase oder ein aktives Fragment davon nach einem der Ansprüche 1 bis 6, wobei die modifizierte Flavinadenindinucleotid-abhängige Glucose-Dehydrogenase oder ein aktives Fragment davon einen Glykosylierungsgrad von <50 % aufweist und/oder das Mw/Mn-Verhältnis <1,02 ist.

8. Isoliertes Polynucleotid, das eine modifizierte Flavinadenindinucleotid-abhängige Glucose-Dehydrogenase oder ein aktives Fragment davon nach einem der Ansprüche 1 bis 6 kodiert, mit der Maßgabe, dass das isolierte Polynucleotid nicht eine modifizierte Flavinadenindinucleotid-abhängige Glucose-Dehydrogenase oder ein aktives Fragment davon kodiert, die/das von der *Aspergillus oryzae*-FAD-GDH-Wildtyp-Sequenz SEQ ID Nr. 2 (Wildtyp) abgeleitet ist, die eine einzige Substitution ausgewählt aus der Gruppe bestehend aus N168K, N168P, N168Y oder N168W aufweist.

9. Expressionsvektor, der das isolierte Polynucleotid nach Anspruch 8 enthält.

10. Wirtszelle, umfassend den Expressionsvektor nach Anspruch 9, die ein isoliertes Polynucleotid beinhaltet, das eine modifizierte Flavinadenindinucleotid-abhängige Glucose-Dehydrogenase oder ein aktives Fragment davon nach einem der Ansprüche 1 bis 6 kodiert, mit der Maßgabe, dass das isolierte Polynucleotid nicht eine modifizierte Flavinadenindinucleotid-abhängige Glucose-Dehydrogenase oder ein aktives Fragment davon kodiert, die/das von der *Aspergillus oryzae*-FAD-GDH-Wildtyp-Sequenz SEQ ID Nr. 2 (Wildtyp) abgeleitet ist, die eine einzige Substitution ausgewählt aus der Gruppe bestehend aus N168K, N168P, N168Y oder N168W aufweist, mit der Maßgabe, dass die Wirtszelle zur Glykosylierung fähig ist, insbesondere zur *N*-Glykosylierung durch Aufweisen von endogenen glykosylierenden Enzymen und dass die Wirtszelle kein *E. coli*-Stamm ist.

11. Verfahren zur Herstellung einer modifizierten Flavinadenindinucleotid-abhängigen Glucose-Dehydrogenase oder eines aktiven Fragments davon, wobei das Verfahren das Kultivieren der Transformante nach Anspruch 10 umfasst.

12. Modifizierte Flavinadenindinucleotid-abhängige Glucose-Dehydrogenase oder ein aktives Fragment davon nach einem der Ansprüche 1 bis 6, erhältlich durch das Verfahren in Anspruch 11 in einer Wirtszelle, die zur Glykosylierung fähig ist.

13. Verfahren zur Erkennung, Bestimmung oder Messung von Glucose in einer *ex vivo* Probe durch Verwendung einer modifizierten Flavinadenindinucleotid-abhängigen Glucose-Dehydrogenase oder eines aktiven Fragments davon nach einem der Ansprüche 1 bis 6 oder Anspruch 12 oder einer Zusammensetzung nach Anspruch 7, wobei die Erkennung, Bestimmung oder Messung das Kontaktieren einer *ex vivo* Probe mit der modifizierten Flavinadenindinucleotid-abhängigen Glucose-Dehydrogenase oder einem aktiven Fragment davon bzw. einer Zusammensetzung umfasst.

14. Verfahren nach Anspruch 13, wobei die Erkennung, Bestimmung oder Messung von Glucose unter Verwendung eines Sensors oder einer Teststreifenvorrichtung durchgeführt wird.

15. Vorrichtung zur Erkennung, Bestimmung oder Messung von Glucose in einer *ex vivo* Probe, umfassend eine modifizierte Flavinadenindinucleotid-abhängige Glucose-Dehydrogenase oder ein aktives Fragment davon nach einem der Ansprüche 1 bis 6 oder Anspruch 12 oder einer Zusammensetzung nach Anspruch 7.

**Revendications**

1. Glucose déshydrogénase dépendante du dinucléotide flavine adénine modifiée qui est glycosylée, choisie parmi le groupe constitué :

(a) d'une glucose déshydrogénase dépendante du dinucléotide flavine adénine modifiée ayant la SEQ ID No: 2 qui est glycosylée, mais au moins un des résidus d'asparagine choisis parmi le groupe constitué de N2, N168 et N346 selon la séquence SEQ ID No: 2 (type sauvage) de FAD-GDH de type sauvage mature d'*Aspergillus oryzae* ayant été substitué par un ou plusieurs acides aminés non appropriés pour la glycosylation et par conséquent éliminant ou inactivant, respectivement, un site de glycosylation potentiel à cette position et
(b) d'une glucose déshydrogénase dépendante du dinucléotide flavine adénine modifiée glycosylée qui présente 80% ou plus d'identité de séquence d'acide aminé avec une glucose déshydrogénase dépendante du dinucléotide flavine adénine constituée de la SEQ ID No: 2, mais au moins un des résidus d'asparagine choisis parmi le groupe constitué de N2, N168 et N346 selon la séquence SEQ ID No: 2 de FAD-GDH de type sauvage mature d'*Aspergillus oryzae* ayant été substitué par un ou plusieurs acides aminés non appropriés pour la glycosylation et par conséquent éliminant ou inactivant, respectivement, un site potentiel de glycosylation à cette position et ledit résidu d'asparagine ayant été substitué par S, P, SP ou D, et

(c) d'un fragment actif d'une glucose déshydrogénase dépendante du dinucléotide flavine adénine modifiée selon (a) ou (b), à condition que dans la FAD-GDH selon (b) ou le fragment selon (c), ladite ou lesdites substitution(s) éliminant ou inactivant ledit(s) site(s) potentiel(s) de glycosylation est/sont préservé(s) par rapport à la glucose déshydrogénase dépendante du dinucléotide flavine adénine modifiée selon (a) et à condition que la glucose déshydrogénase dépendante du dinucléotide flavine adénine selon (b) ou le fragment selon (c) présente au moins 80% de l'activité enzymatique de la FAD-GDH selon (a) et au moins 80% de la stabilité de température dans des conditions sèches de la FAD-GDH selon (a), l'expression « présente une stabilité de température dans des conditions sèches » signifiant l'activité résiduelle de la glucose déshydrogénase dépendante du dinucléotide flavine adénine modifiée lyophilisée (FAD-GDH) elle-même et lorsque comprise dans une composition lyophilisée, est calculée et comparée au lyophilisat non soumis à une contrainte après :

1) lyophilisation et incubation de l'enzyme lyophilisée à 80°C pendant 8 jours sur tamis moléculaire (3A, MS551, Grace).

2. Glucose déshydrogénase dépendante du dinucléotide flavine adénine modifiée ou fragment actif de celle-ci selon la revendication 1, ladite glucose déshydrogénase dépendante du dinucléotide flavine adénine modifiée présentant une stabilité de température améliorée dans des conditions sèches par rapport à la FAD-GDH glycosylée selon la SEQ ID No: 1, ladite FAD-GDH selon la SEQ ID No: 1 pouvant être obtenue par expression dans *Aspergillus oryzae*.

3. Glucose déshydrogénase dépendante du dinucléotide flavine adénine modifiée ou fragment actif de celle-ci selon la revendication 1 ou 2, ladite glucose déshydrogénase dépendante du flavine adénine dinucléotide modifiée ou un fragment actif de celle-ci présentant un degré de glycosylation qui est < 50 %, et/ou le rapport Mw/Mn est < 1,02.

4. Glucose déshydrogénase dépendante du dinucléotide flavine adénine modifiée ou fragment actif de celle-ci selon une quelconque des revendications 1 à 3, seulement un des résidus d'asparagine choisis parmi le groupe constitué de N2, N168 et N346 ayant été substitué par un ou plusieurs acides aminés non appropriés pour la glycosylation et par conséquent éliminant ou inactivant, respectivement, un site potentiel de glycosylation à cette position.

5. Glucose déshydrogénase dépendante du dinucléotide flavine adénine modifiée ou fragment actif de celle-ci selon une quelconque des revendications 1 à 4, ladite glucose déshydrogénase dépendante du dinucléotide flavine adénine modifiée ou un fragment actif de celle-ci dérivé de la séquence SEQ ID No: 2 (type sauvage) de FAD-GDH de type sauvage d'*Aspergillus oryzae* ayant une ou plusieurs des substitutions suivantes N2S, N168P, N168SP, et N346D.

6. Glucose déshydrogénase dépendante du dinucléotide flavine adénine modifiée ou fragment actif de celle-ci selon une quelconque des revendications 1 à 5, ladite glucose déshydrogénase dépendante du dinucléotide flavine adénine modifiée ou un fragment actif de celle-ci dérivé de la séquence SEQ ID No: 2 (type sauvage) de FAD-GDH de type sauvage d'*Aspergillus oryzae* ayant la substitution suivante N2S et étant une FAD-GDH selon SEQ ID No: 3 ou un fragment actif de celle-ci (SEQ ID No: 3).

7. Composition comprenant une glucose déshydrogénase dépendante du dinucléotide flavine adénine modifiée ou un fragment actif de celle-ci selon une quelconque des revendications 1 à 6, ladite glucose déshydrogénase dépendante du dinucléotide flavine adénine modifiée ou un fragment actif de celle-ci présentant un degré de glycosylation qui est < 50 %, et/ou le rapport Mw/Mn est < 1,02.

8. Polynucléotide isolé codant une glucose déshydrogénase dépendante du dinucléotide flavine adénine modifiée ou un fragment actif de celle-ci selon une quelconque des revendications 1 à 6 à condition que ledit polynucléotide isolé ne code pas pour une glucose déshydrogénase dépendante du dinucléotide flavine adénine modifiée ou un fragment actif de celle-ci dérivé de la séquence SEQ ID No: 2 (type sauvage) de FAD-GDH de type sauvage d'*Aspergillus oryzae* ayant une seule substitution choisie parmi le groupe constitué de N168K, N168P, N168Y, ou N168W.

9. Vecteur d'expression contenant le polynucléotide isolé selon la revendication 8.

**10.** Cellule hôte comprenant le vecteur d'expression selon la revendication 9, incluant un polynucléotide isolé codant pour une glucose déshydrogénase dépendante du dinucléotide flavine adénine modifiée ou un fragment actif de celle-ci selon une quelconque des revendications 1 à 6 à condition que ledit polynucléotide isolé ne code pas pour une glucose déshydrogénase dépendante du dinucléotide flavine adénine modifiée ou un fragment actif de celle-ci dérivé de la séquence SEQ ID No: 2 (type sauvage) de FAD-GDH de type sauvage d'*Aspergillus oryzae* ayant une seule substitution choisie parmi le groupe constitué de N168K, N168P, N168Y, ou N168W, à condition que ladite cellule hôte soit capable de glycosylation, en particulier pour la glycosylation liée à *N*, par des enzymes de glycosylation endogènes, et ladite cellule hôte n'étant pas une souche d'E. coli.

**11.** Procédé de production d'une glucose déshydrogénase dépendante du dinucléotide flavine adénine modifiée ou d'un fragment actif de celle-ci, le procédé comprenant la culture du transformant selon la revendication 10.

**12.** Glucose déshydrogénase dépendante du dinucléotide flavine adénine modifiée ou fragment actif de celle-ci selon une quelconque des revendications 1 à 6 pouvant être obtenu par le procédé selon la revendication 11 dans une cellule hôte capable de glycosylation.

**13.** Méthode de détection, de détermination ou de mesure du glucose dans un échantillon *ex vivo* à l'aide d'une glucose déshydrogénase dépendante du dinucléotide flavine adénine modifiée ou d'un fragment actif de celle-ci selon une quelconque des revendications 1 à 6, ou la revendication 12 ou une composition selon la revendication 7, ladite détection, détermination ou mesure comprenant la mise en contact d'un échantillon *ex vivo* avec ladite glucose déshydrogénase dépendante du dinucléotide flavine adénine modifiée ou un fragment actif ou une composition, respectivement.

**14.** Méthode selon la revendication 13, ladite détection, détermination ou mesure du glucose étant effectuée à l'aide d'un capteur ou d'un dispositif à bandelettes de test.

**15.** Dispositif de détection, de détermination ou de mesure du glucose dans un échantillon *ex vivo* comprenant une glucose déshydrogénase dépendante du dinucléotide flavine adénine modifiée ou un fragment actif de celle-ci selon une quelconque des revendications 1 à 6, ou la revendication 12 ou une composition selon la revendication 7.

Fig. 1:

Fig. 2:

**Fig. 3:**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2004058958 A1
- WO 2006101239 A1
- US 7662600 B2
- US 2008220460 A1
- JP 2010239969 A
- US 20090259024 A1
- US 20090155848 A
- US 020080090278 A1
- US 20080020426 A1
- US 20080014612 A1
- US 20080014611 A1
- US 20080003628 A1
- US 7871805 B2
- US 7741100 B2
- US 7655130 B2
- US 7553649 B2
- US 7494794 B2

### Non-patent literature cited in the description

- **ALTSCHUL, S.F. et al.** *J. Mol. Biol.,* 1990, vol. 215, 403
- **ALTSCHUL, S.F. et al.** *Nucleic Acid Res.,* 1997, vol. 25, 3389-3402
- **SAMBROOK et al.** Molecular Cloning: A Laboratory Manual. Cold Spring Habor Laboratory Press, 1989
- **J. SAMBROOK ; D. W. RUSSELL.** Molecular Cloning: a laboratory manual. Cold Spring Harbor, 2001
- **D'COSTA, E. J. et al.** *Biosensors,* 1986, vol. 2, 71-87
- **LAURINAVICIUS, V. et al.** *Analytical Letters,* 1999, vol. 32, 299-316
- **LAURINAVICIUS, V. et al.** *Monatshefte fuer Chemie,* 1999, vol. 130, 1269-1281
- **MALINAUSKAS, A. et al.** *Sensors and Actuators, B: Chemical,* 2004, vol. 100, 395-402
- **AUSUBEL, F. et al.** Current protocols in molecular biology. Wiley, 1994
- **HANEY, MAX.** Basics of GPC/SEC with threefold detection. *LaborPraxis,* 2004, vol. 28, 50-53
- **WANDA K. HARTMANN et al.** Characterization and analysis of thermal denaturation of antibodies by size exclusion high-performance liquid chromatography with quadruple detection. *Analytical Biochemistry,* 2004, vol. 325, 227-239
- **HEINZMANN, GERHARD.** *Tartsch, Bernd GPC/SEC - three eyes can see more GIT Spezial Separation,* 2007, vol. 27, 21-24
- **L. MICHAELIS ; M.L. MENTEN.** *Die Kinetik der Invertinwirkung Biochem. Z.,* 1913, vol. 49, 333-369